# EUROPEAN PATENT SPECIFICATION

(11) **EP 2 450 455 B1**
(45) Date of publication and mention of the grant of the patent: **16.03.2016**
(21) Application number: 10794192.4
(22) Date of filing: 30.06.2010
(51) Int. Cl.: C12Q 1/68, C12N 15/09, G01N 33/53, G01N 37/00

(54) **METHOD FOR DETERMINING PRESENCE OR ABSENCE OF EPITHELIAL CANCER-ORIGIN CELL IN BIOLOGICAL SAMPLE, AND KIT THEREFOR**
VERFAHREN ZUR BESTIMMUNG DER PRÄSENZ ODER ABSENZ EPITHELIALKREBSBILDENDER ZELLEN IN EINER BIOLOGISCHEN PROBE SOWIE KIT DAFÜR
PROCÉDÉ PERMETTANT DE DÉTERMINER SI DES CELLULES ÉPITHÉLIALES D'ORIGINE CANCÉREUSES SONT PRÉSENTES OU PAS DANS UN ÉCHANTILLON BIOLOGIQUE ET NÉCESSAIRE ASSOCIÉS

(30) Priority: 30.06.2009 JP 2009155572
(43) Date of publication of application: 09.05.2012
(73) Proprietor: Sysmex Corporation, Kobe-shi, Hyogo 651-0073 (JP)
(72) Inventor: TAI, Kaya, Kobe-shi Hyogo 651-0073 (JP); KAJITA, Masahiro, Kobe-shi Hyogo 651-0073 (JP); TASHIMA, Yoshihiko, Kobe-shi Hyogo 651-0073 (JP); SAKAI, Ayako, Kobe-shi Hyogo 651-0073 (JP)
(74) Representative: Paemen, Liesbet R.J.
(86) International application number: PCT/JP2010/061149
(87) International publication number: WO 2011/002024

(56) References cited:
- WO-A1-2007/019670
- WO-A2-2005/059172
- WO-A2-2005/123945
- WO-A2-2006/008128
- WO-A2-2009/037635
- WO-A2-2009/037635
- DATABASE EMBL [Online] 24 September 2001 (2001-09-24), "Homo sapiens, protocadherin gamma subfamily A, 10, clone IMAGE:4865718, mRNA.", XP002685813, retrieved from EBI accession no. EM_HTC:BC014509 Database accession no. BC014509
- ESTELLER M ET AL: "A GENE HYPERMETHYLATION PROFILE OF HUMAN CANCER", CANCER RESEARCH, AMERICAN ASSOCIATION FOR CANCER RESEARCH, US, vol. 61, 15 April 2001 (2001-04-15), pages 3225-3229, XP002982492, ISSN: 0008-5472
- HERMAN J G ET AL: "INACTIVATION OF THE CDKN2/P16/MTS1 GENE IS FREQUENTLY ASSOCIATED WITH ABERRANT DNA METHYLATION IN ALL COMMON HUMAN CANCERS", CANCER RESEARCH, AMERICAN ASSOCIATION FOR CANCER RESEARCH, US, vol. 55, no. 20, 15 October 1995 (1995-10-15), pages 4525-4530, XP001109420, ISSN: 0008-5472
- MEI Y. YU ET AL: "Hypermethylation of the tumor suppressor gene RASSFIA and frequent concomitant loss of heterozygosity at 3p21 in cervical cancers", INTERNATIONAL JOURNAL OF CANCER, vol. 105, no. 2, 10 June 2003 (2003-06-10) , pages 204-209, XP55018294, ISSN: 0020-7136, DOI: 10.1002/ijc.11051
- SHAMES DAVID S ET AL: "A genome-wide screen for promoter methylation in lung cancer identifies novel methylation markers for multiple malignancies", PLOS MEDICINE, PUBLIC LIBRARY OF SCIENCE, US, vol. 3, no. 12, 1 December 2006 (2006-12-01), XP002527542, ISSN: 1549-1676, DOI: 10.1371/JOURNAL.PMED.0030486
- BURBEE D G ET AL: "EPIGENETIC INACTIVATION OF RASSF1A IN LUNG AND BREAST CANCERS AND MALIGNANT PHENOTYPE SUPPRESSION", JOURNAL OF THE NATIONAL CANCER INSTITUTE, OXFORD UNIVERSITY PRESS, GB, vol. 93, no. 9, 2 May 2001 (2001-05-02), pages 691-699, XP008006020, ISSN: 0027-8874, DOI: 10.1093/JNCI/93.9.691
- DUNWELL THOMAS ET AL: "A Genome-wide screen identifies frequently methylated genes in haematological and epithelial cancers", MOLECULAR CANCER, BIOMED CENTRAL, LONDON, GB, vol. 9, no. 1, 25 February 2010 (2010-02-25), page 44, XP021067996, ISSN: 1476-4598
- MIYAMOTO K. ET AL.: 'Diagnostic and therapeutic applications of epigenetics.' JPN. J. CLIN. ONCOL. vol. 35, 2005, pages 293 - 301, XP007907854
- YANG X. ET AL.: 'DNA methylation in breast cancer.' ENDOCR. RELAT. CANCER vol. 8, 2001, pages 115 - 127, XP007907855
- HOQUE M.O. ET AL.: 'Detection of aberrant methylation of four genes in plasma DNA for the detection of breast cancer.' J. CLIN. ONCOL. vol. 24, 2006, pages 4262 - 4269, XP002500186
- P. Novak ET AL: "Agglomerative Epigenetic Aberrations Are a Common Event in Human Breast Cancer", Cancer research, vol. 68, no. 20, 15 October 2008 (2008-10-15), pages 8616-8625, XP055130800, ISSN: 0008-5472, DOI: 10.1158/0008-5472.CAN-08-1419

## Description

### TECHNICAL FIELD

The present invention relates to a method for determining the presence or absence of an epithelial cancer-derived cell in a biological sample as well as a kit therefor.

### BACKGROUND ART

It has been known that various methods such as blood tests and image inspections, e.g. X-ray examination are used for detection of epithelial cancers. For example, currently known detection techniques for the epithelial cancer breast cancer, are image inspections such as mammography and MRI. These image inspections contribute to a reduction in mortality of breast cancer by detecting breast cancer at early stages.

These image inspections, however, have a number of drawbacks in that they require experienced skills, they may be uncomfortable for patients and they are expensive.

In order to solve the above problems of image inspections, research has been recently carried out on cancer detection methods based on genetic information. Such cancer detection methods include, for example, cancer detection methods based on gene methylation information. In these methods, CpG sites (5'-(CG)-3') in specific genes are used as molecular markers, the methylation status of the molecular markers is analyzed, and cancer is detected based on the obtained information.

Studies have been also carried out on methods for detection of breast cancer utilizing gene methylation analysis. In those methods, CpG sites in specific genes are used as molecular markers, the methylation status of the molecular markers is analyzed, and the obtained results are used for detection of breast cancer. For example, Non-patent Literature 1 discloses that CpG sites in the promoter regions of *breast cancer 1* (*BRCA1), cadherin 13* (*CDH13*) and *serum deprivation response factor (sdr)-related gene product that binds to c-kinase* (*SRBC*) are not methylated in normal cells but methylated in breast cancer cells. Patent Literature 1 discloses that breast cell proliferative diseases can be diagnosed by analyzing the methylation status of the genes *breast cancer 2* (*BRCA2*) and *protocadherin 7* (*PCDH7*). Novak et al (Cancer Res., 2008, 68(20) 8616-8625) disclosed hypermethylation in the PCDH gene cluster in breast cancer cells compared with normal breast cells

### Citation List

### Patent Literature

Patent Literature 1: Japanese Translation of PCT Application No. 2008-506407

### Non-patent Literature

Non-patent Literature 1: Manel Esteller. Nat Rev Genet, 2007 Apr; 8(4): 286-98

### SUMMARY OF INVENTION

### Technical Problem

As described above, some genes have been reported to be abnormally methylated in cancer. However, much better novel molecular markers are desired that allow detection of cancer by gene methylation analyses.

An object of the present invention is to provide a method for determining the presence or absence of an epithelial cancer-derived cell based on results obtained by analyzing the methylation status of DNA extracted from a biological sample with a novel molecular marker that allows the determination of the presence or absence of the epithelial cancer-derived cell.

Another object of the present invention is to provide a kit for determining the presence or absence of an epithelial cancer-derived cell by DNA methylation analysis using the novel molecular marker.

### Solution to Problem

The present inventors have found that the methylation status of the CpG site included in the regions represented by base sequences SEQ ID NOs: 1, 2, 3 and 4 in human genomic DNA is different between particular epithelial cancer cell types and normal cells.

Thus, the present disclosure provides a method for determining the presence or absence of an epithelial cancer-derived cell in a biological sample obtained from a subject comprising the steps of extracting DNA from the biological sample, analyzing the methylation status of a CpG site located in at least one region represented by base sequences SEQ ID NOs: 1, 2, 3 and 4 in the DNA obtained from the extraction step, and determining the presence or absence of the epithelial cancer-derived cell in the biological sample based on an analysis result obtained from the step of analyzing.

The present disclosure also provides a molecular marker for the determination of the presence or absence of an epithelial cancer-derived cell by methylation analysis, which is at least one CpG site located in at least one region represented by base sequences SEQ ID NOs: 1, 2, 3 and 4.

The present disclosure also provides a kit for determining the presence or absence of an epithelial cancer-derived cell in a biological sample obtained from a subject comprising a non-methylated cytosine conversion agent that converts non-methylated cytosine in DNA extracted from the biological sample to a different base, and a primer set for detecting the methylation of at least one CpG site located in a region represented by base sequence SEQ ID NO: 1, 2, 3 or 4 by methylation-specific PCR.

The present invention is defined by the appended claims.

### Effects of Invention

The present invention can provide a method for determining the presence or absence of particular epithelial cancer-derived cell types in a biological sample obtained from a subject by analyzing the methylation status of the molecular marker of the present invention in DNA extracted from the biological sample.

The present invention also provides a kit which can be used for the above-described determination method.

### BRIEF DESCRIPTION OF DRAWINGS

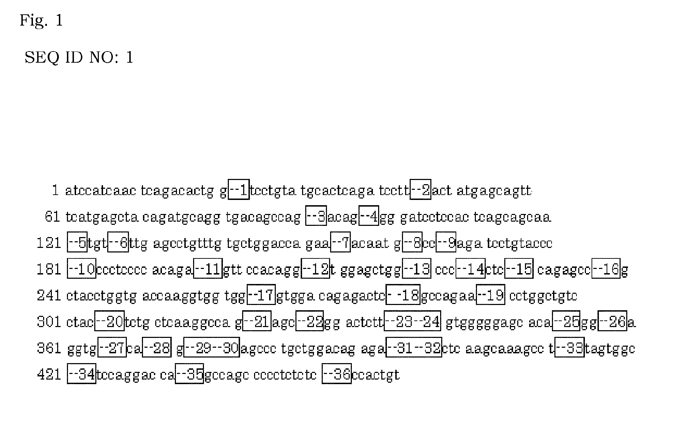

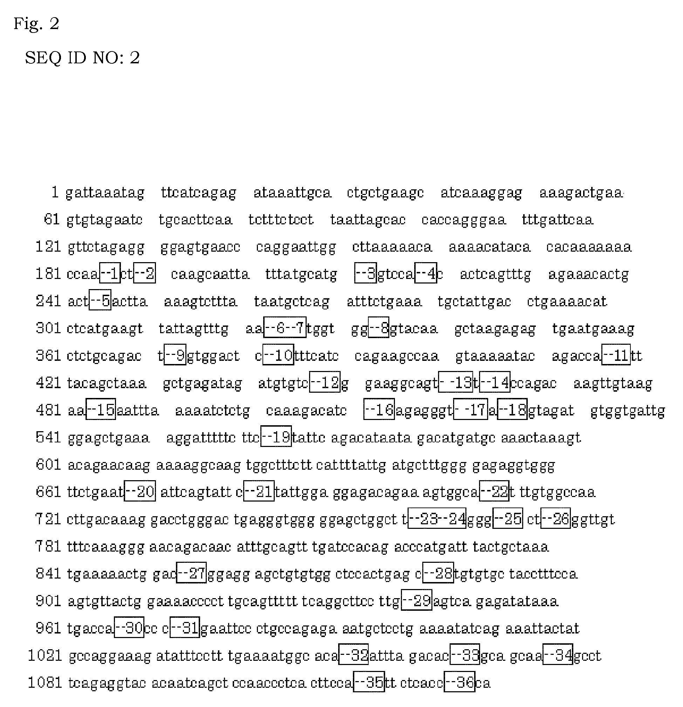

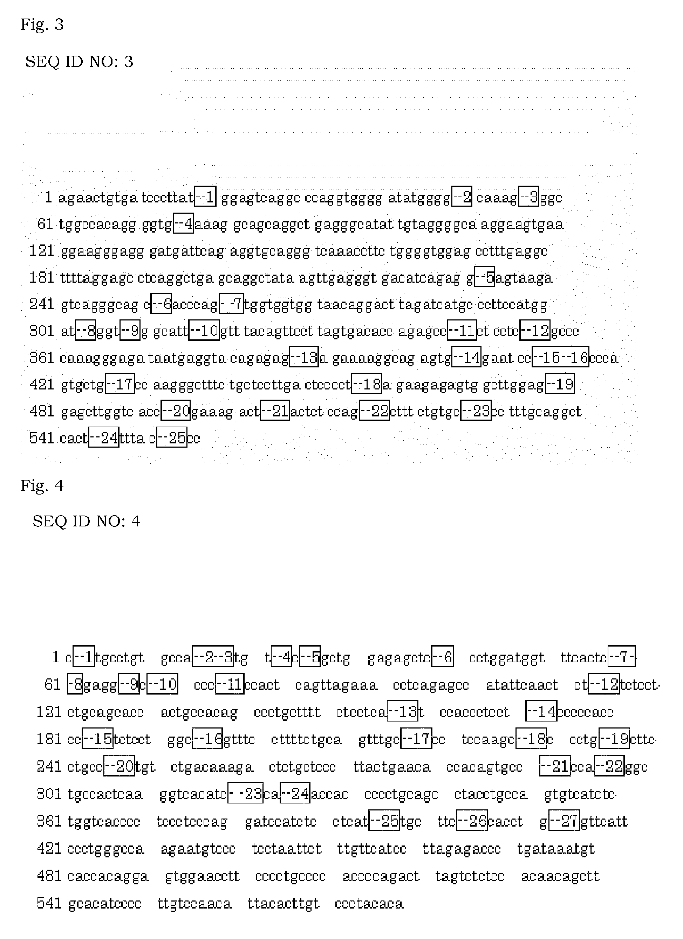

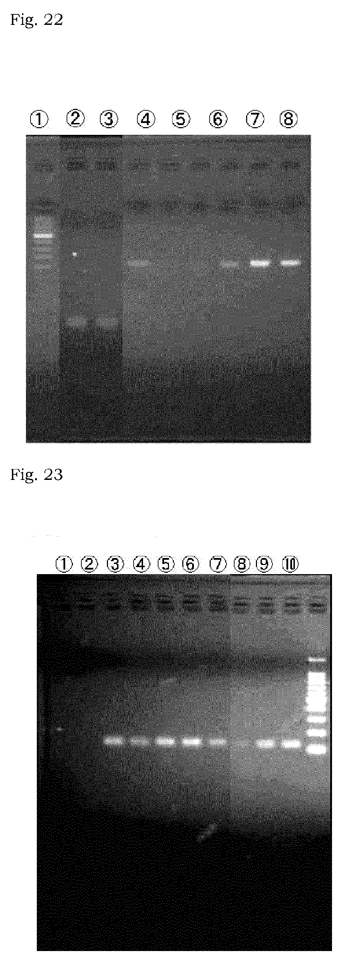

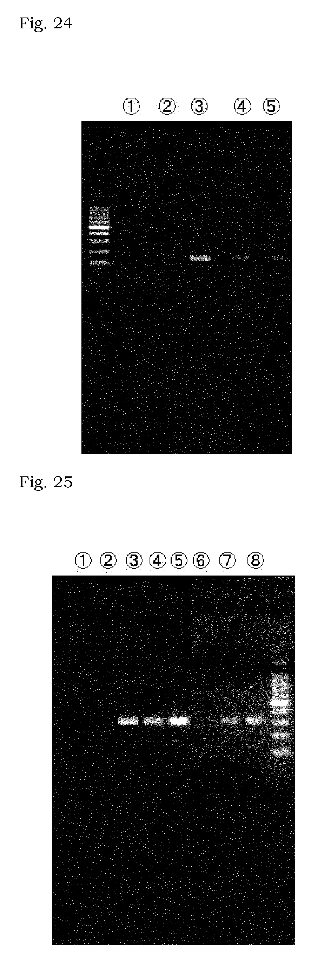

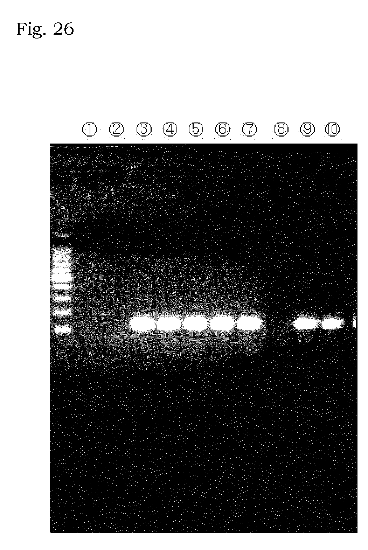

Fig 1 is the sequence showing the positions of CpG sites included in the region represented by the base sequence SEQ ID NO: 1;
   "- -" denotes CG; the number following "- -" denotes the order of the CpG site from the 5' end of the base sequence.
Fig 2 is the sequence showing the positions of CpG sites included in the region represented by the base sequence SEQ ID NO: 2; "- -" denotes CG; the number following "- -" denotes the order of the CpG site from the 5' end of the base sequence. Fig 3 is the sequence showing the positions of CpG sites included in the region represented by the base sequence SEQ ID NO: 3;
   "- -" denotes CG; the number following "- -" denotes the order of the CpG site from the 5' end of the base sequence.
Fig 4 is the sequence showing the positions of CpG sites included in the region represented by the base sequence SEQ ID NO: 4;
   "- -" denotes CG; the number following "- -" denotes the order of the CpG site from the 5' end of the base sequence.
Fig. 5 is a table showing the presence or absence of methylation of the respective CpG sites included in the region represented by the base sequence SEQ ID NO: 1;
   In this Table, the numbers shown in the row of "CpG site" correspond to the numbers shown in the base sequence in Fig. 1.
Fig. 6 is a table showing the presence or absence of methylation of the respective CpG sites included in the region represented by the base sequence SEQ ID NO: 2;
   In this Table, the numbers shown in the row of "CpG site" correspond to the numbers shown in the base sequence in Fig. 2. Fig. 7 is a table showing the presence or absence of methylation of the respective CpG sites included in the region represented by the base sequence SEQ ID NO: 3;
   In this Table, the numbers shown in the row of "CpG site" correspond to the numbers shown in the base sequence in Fig. 3.

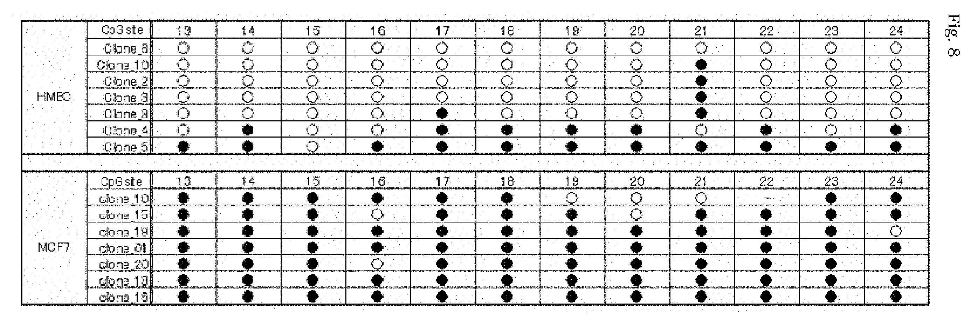

Fig. 8 is a table showing the presence or absence of methylation of the respective CpG sites included in the region represented by the base sequence SEQ ID NO: 4;
   In this Table, the numbers shown in the row of "CpG site" correspond to the numbers shown in the base sequence in Fig. 4.

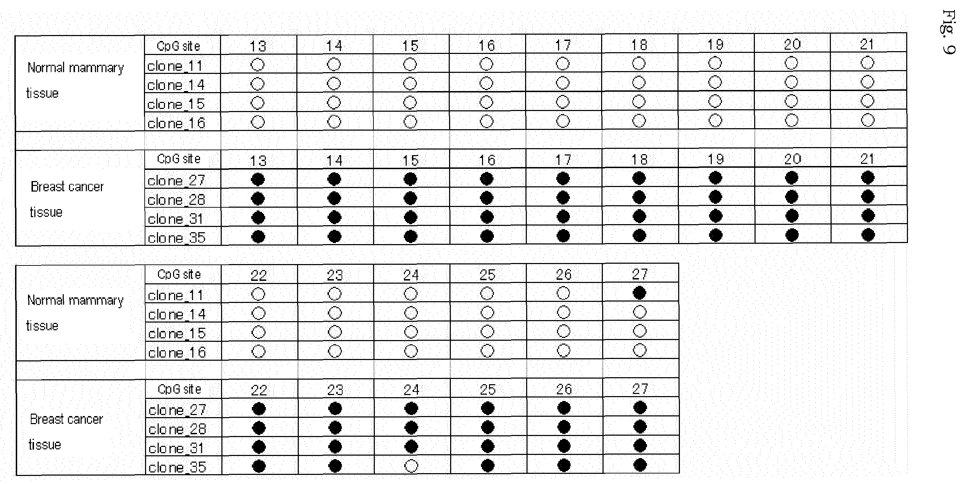

Fig. 9 is a table showing the presence or absence of methylation of the respective CpG sites included in the region represented by the base sequence SEQ ID NO: 4;
   In this Table, the numbers shown in the row of "CpG site" correspond to the numbers shown in the base sequence in Fig. 4.

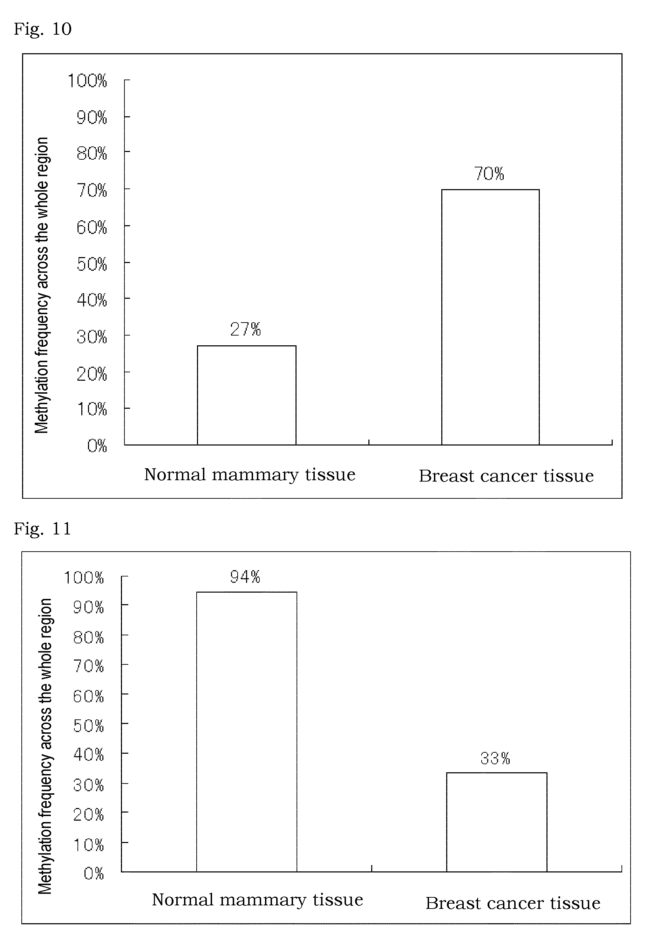

Fig. 10 is a graph representing the result of methylation frequency analysis in the region represented by the base sequence SEQ ID NO: 1;
Fig. 11 is a graph representing the result of methylation frequency analysis in the region represented by the base sequence SEQ ID NO: 2;

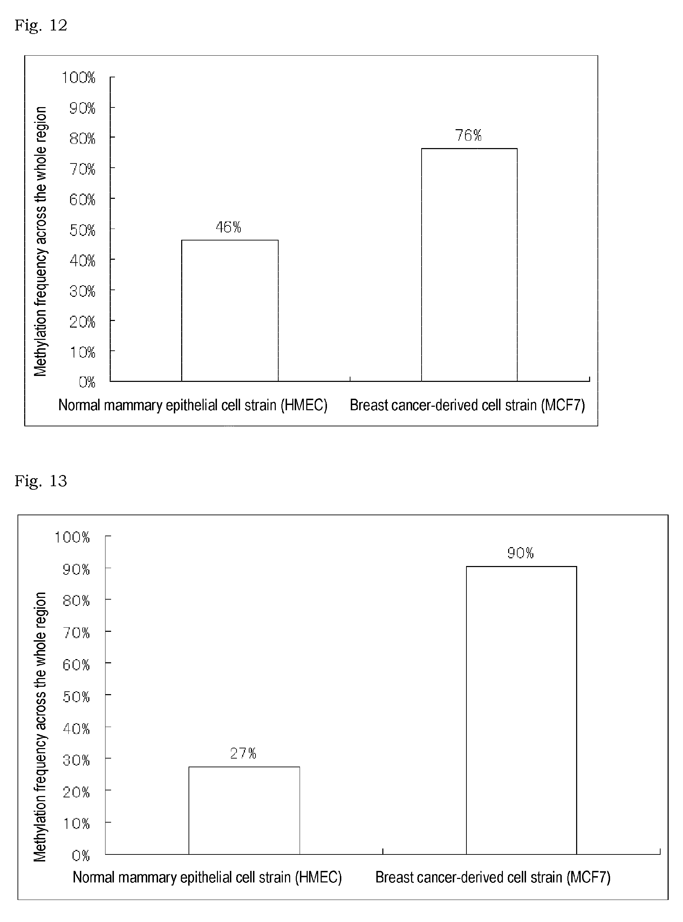

Fig. 12 is a graph representing the result of methylation frequency analysis in the region represented by the base sequence SEQ ID NO: 3;
Fig. 13 is a graph representing the result of methylation frequency analysis in the region represented by the base sequence SEQ ID NO: 4;

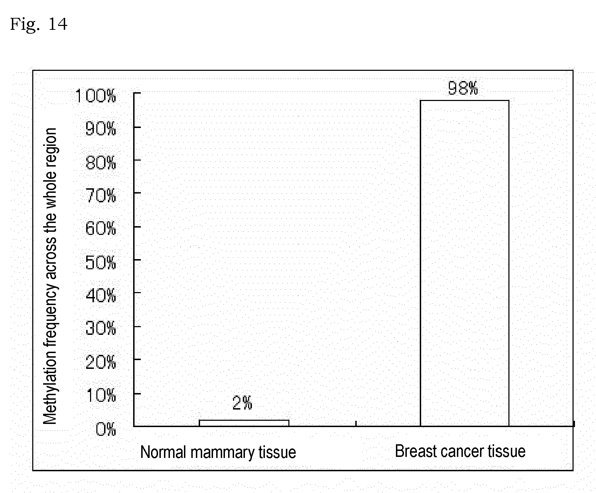

Fig. 14 is a graph representing the result of methylation frequency analysis in the region represented by the base sequence SEQ ID NO: 4;

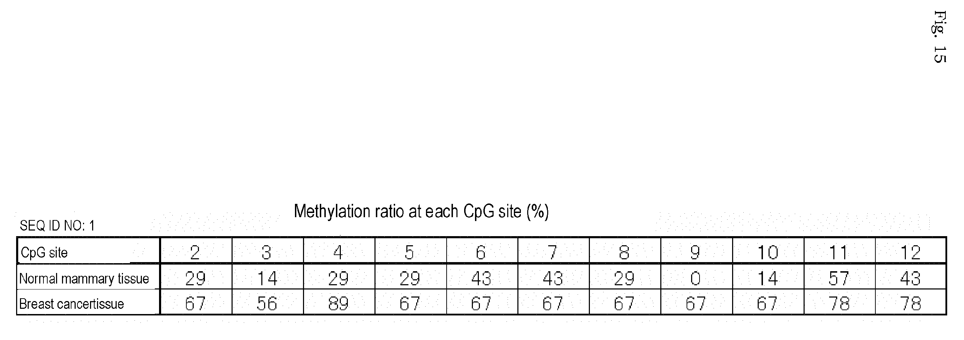

Fig. 15 is a table representing the methylation ratio of the respective CpG sites included in the region represented by the base sequence SEQ ID NO: 1;
   In this Table, the numbers shown in the row of "CpG site" correspond to the numbers shown in the base sequence in Fig. 1.

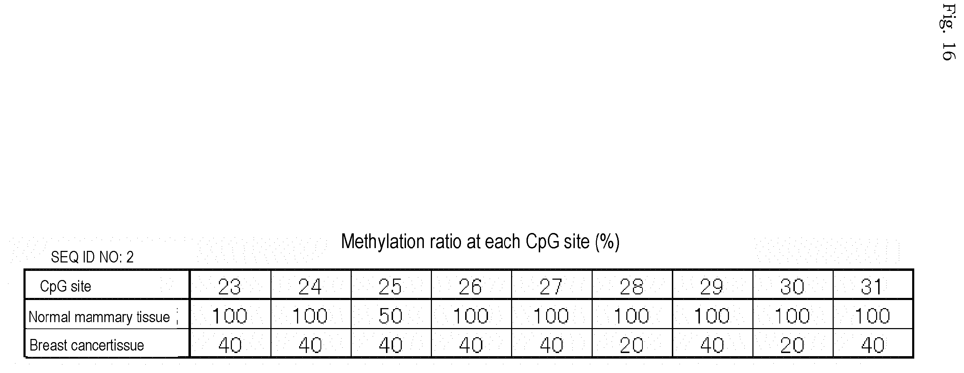

Fig. 16 is a table representing the methylation ratio of the respective CpG sites included in the region represented by the base sequence SEQ ID NO: 2;
   In this Table, the numbers shown in the row of "CpG site" correspond to the numbers shown in the base sequence in Fig. 2.

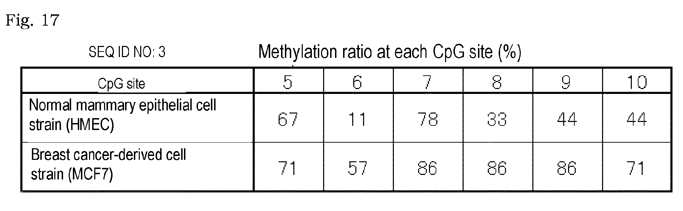

Fig. 17 is a table representing the methylation ratio of the respective CpG sites included in the region represented by the base sequence SEQ ID NO: 3;
   In this Table, the numbers shown in the row of "CpG site" correspond to the numbers shown in the base sequence in Fig. 3.

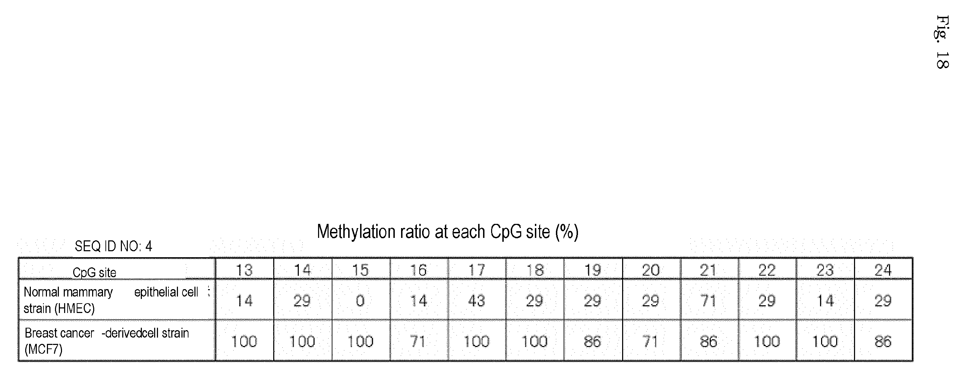

Fig. 18 is a table representing the methylation ratio of the respective CpG sites included in the region represented by the base sequence SEQ ID NO: 4;
   In this Table, the numbers shown in the row of "CpG site" correspond to the numbers shown in the base sequence in Fig. 4.

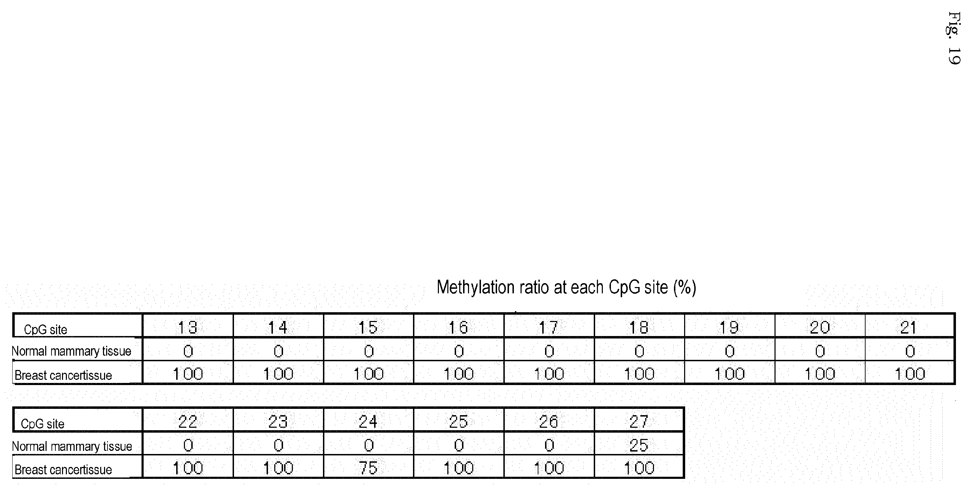

Fig. 19 is a table representing the methylation ratio of the respective CpG sites included in the region represented by the base sequence SEQ ID NO: 4;
   In this Table, the numbers shown in the row of "CpG site" correspond to the numbers shown in the base sequence in Fig. 4.

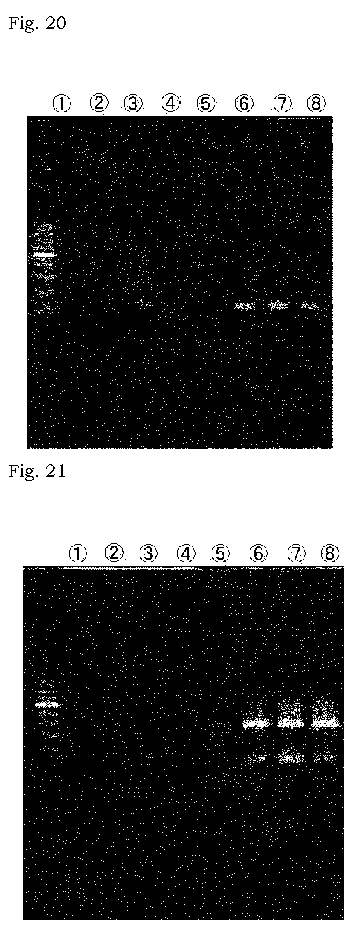

Fig. 20 is an electrophoresis photo for verifying the presence or absence of the amplified product after methylation specific PCR of the region represented by the base sequence SEQ ID NO: 1 (Methylation specific PCR analysis of SEQ ID NO: 1);
   Lane 1: PCR reaction solution
   Lane 2: PCR reaction solution + water
   Lane 3: Normal mammary tissue sample A
   Lane 4: Normal mammary tissue sample B
   Lane 5: Normal mammary tissue sample C
   Lane 6: Breast cancer tissue sample D
   Lane 7: Breast cancer tissue sample E
   Lane 8: Breast cancer tissue sample F
Fig. 21 is an electrophoresis photo for verifying the presence or absence of the amplified product after methylation specific PCR of the region represented by the base sequence SEQ ID NO: 3 (Methylation specific PCR analysis of SEQ ID NO: 3);
   Lane 1: PCR reaction solution
   Lane 2: PCR reaction solution + water
   Lane 3: Normal mammary tissue sample A
   Lane 4: Normal mammary tissue sample B
   Lane 5: Normal mammary tissue sample C
   Lane 6: Breast cancer tissue sample D
   Lane 7: Breast cancer tissue sample E
   Lane 8: Breast cancer tissue sample F
Fig. 22 is an electrophoresis photo for verifying the presence or absence of the amplified product after methylation specific PCR of the region represented by the base sequence SEQ ID NO: 4 (Methylation specific PCR analysis of SEQ ID NO: 4);
   Lane 1: PCR reaction solution
   Lane 2: PCR reaction solution + water
   Lane 3: Normal mammary tissue sample A
   Lane 4: Normal mammary tissue sample B
   Lane 5: Normal mammary tissue sample C
   Lane 6: Breast cancer tissue sample D
   Lane 7: Breast cancer tissue sample E
   Lane 8: Breast cancer tissue sample F
Fig. 23 is an electrophoresis photo for verifying the presence or absence of the amplified product after methylation specific PCR of the region represented by the base sequence SEQ ID NO: 1 (Methylation specific PCR analysis of SEQ ID NO: 1);
   Lane 1: PCR reaction solution
   Lane 2: PCR reaction solution + water
   Lane 3: Large bowel cancer cell strain HCT16
   Lane 4: Gastric cancer cell strain KATO3
   Lane 5: Cervical cancer cell strain C33A
   Lane 6: Cervical cancer cell strain SiHa
   Lane 7: Cervical cancer cell strain Hela
   Lane 8: Normal large bowel tissue sample
   Lane 9: Large bowel cancer tissue sample A
   Lane 10: Large bowel cancer tissue sample B
Fig. 24 is an electrophoresis photo for verifying the presence or absence of the amplified product after methylation specific PCR of the region represented by the base sequence SEQ ID NO: 2 (Methylation specific PCR analysis of SEQ ID NO: 2);
   Lane 1: PCR reaction solution
   Lane 2: PCR reaction solution + water
   Lane 3: Normal large bowel tissue sample
   Lane 4: Large bowel cancer tissue sample A
   Lane 5: Large bowel cancer tissue sample B
Fig. 25 is an electrophoresis photo for verifying the presence or absence of the amplified product after methylation specific PCR of the region represented by the base sequence SEQ ID NO: 3 (Methylation specific PCR analysis of SEQ ID NO: 3);
   Lane 1: PCR reaction solution
   Lane 2: PCR reaction solution + water
   Lane 3: Large bowel cancer cell strain HCT16
   Lane 4: Cervical cancer cell strain C33A
   Lane 5: Cervical cancer cell strain SiHa
   Lane 6: Normal large bowel tissue sample
   Lane 7: Large bowel cancer tissue sample A
   Lane 8: Large bowel cancer tissue sample B
Fig. 26 is an electrophoresis photo for verifying the presence or absence of the amplified product after methylation specific PCR of the region represented by the base sequence SEQ ID NO: 4 (Methylation specific PCR analysis of SEQ ID NO: 4);
   Lane 1: PCR reaction solution
   Lane 2: PCR reaction solution + water
   Lane 3: Large bowel cancer cell strain HCT16
   Lane 4: Gastric cancer cell strain KATO3
   Lane 5: Cervical cancer cell strain C33A
   Lane 6: Cervical cancer cell strain SiHa
   Lane 7: Cervical cancer cell strain Hela
   Lane 8: Normal large bowel tissue sample
   Lane 9: Large bowel cancer tissue sample A
   Lane 10: Large bowel cancer tissue sample B

### DESCRIPTION OF EMBODIMENTS

The present method, molecular marker and kit for determining the presence or absence of an epithelial cancer-derived cell are now described hereinafter.

In the present disclosure epithelial cancer means malignant tumor derived from epithelial tissues. The malignant tumor derived from epithelial tissues may include malignant tumors developed in different sites e.g. breast, lung, liver, stomach, large bowel, pancreas, uterus, testicle, ovary, thyroid and the like. Epithelial cancer may specifically include breast cancer, lung cancer, liver cancer, gastric cancer, large bowel cancer, pancreatic cancer, uterine cancer, testicular cancer, ovarian cancer, thyroid cancer and the like.

In the present invention, a biological sample is not specifically limited so long as it contains DNA. The specific biological sample may include, for example, blood, serum, lymph fluid, urine, nipple discharge, tissues obtained by operations and biopsies and the like. A sample obtained by culturing cells or tissues obtained from a subject can also be a biological sample.

In the present invention, DNA can be extracted by any well-known methods that can release DNA contained in a biological sample into solution. Well-known DNA extraction methods may include, for example, a method in which a biological sample is mixed with a surfactant capable of solubilizing the biological sample and the mixture is subjected to a physical treatment (stirring, homogenization, ultrasonication and the like). In this case, it is preferable that cell debris and the like are precipitated by centrifugation after the physical treatment and the supernatant containing DNA is used for the analysis described hereinafter. DNA can be extracted using commercially available kits.

The base sequences represented by SEQ ID NOs: 1 to 4 are partial regions in human genomic DNA. The base sequences of human genomic DNA can be obtained from public databases (e.g. GenBank from National Center for Biotechnology Information (NCBI)).

The base sequence represented by SEQ ID NO: 1 is a part of the promoter region of the gene *protocadherin gamma subfamily A, 10 (PCDHGA10).* This gene encodes for the calcium-dependent intercellular adhesion molecule belonging to cadherin superfamily. It is suggested that the *PCDHGA10* gene may be associated with the constitution and maintenance of the connection between specific neurons in the brain.

SEQ ID NO: 2 is a part of the promoter region of the gene *protocadherin beta 6 (PCDHB6).* This gene belongs to, as *PCDHGA10,* cadherin superfamily.

SEQ ID NO: 3 is a part of an intron of the gene *ladybird homeobox 2 (LBX2).* This gene is a homologous gene of *Drosphila* ladybird gene, and its association with Alstrom syndrome has been studied.

SEQ ID NO: 4 is the base sequence included in chromosome 1 of human genomic DNA. Its function is not yet elucidated.

In the present invention, methylation of a CpG site denotes methylation of a cytosine base at its 5- or 6-position of the CpG site (5'-(CG)-3') in a DNA base sequence.

In the present disclosure "analyzing the methylation status" means to analyze the presence or absence of methylation of a CpG site located in a region represented by a base sequence to be analyzed, or to analyze the methylation frequency of a CpG site located in the base sequence, wherein "a base sequence to be analyzed" is at least one base sequence selected from the base sequences SEQ ID NOs: 1 to 4.

The above "presence or absence of methylation" means whether or not a cytosine of at least one CpG site located in a region represented by a base sequence to be analyzed is methylated.

The above "methylation frequency" means the ratio of the number of methylated CpG site(s) relative to total or predetermined CpG sites located in a region represented by a base sequence to be analyzed. Methylation frequency may be analyzed for each base sequence among SEQ ID NOs: 1 to 4, or analyzed simultaneously for multiple base sequences selected from SEQ ID NOs: 1 to 4.

According to the present disclosure the CpG site to be analyzed for its methylation status is preferably selected from the following:
the 2nd to 12th CpG sites from the 5' end in the region represented by the base sequence SEQ ID NO: 1;
the 23rd to 31st CpG sites from the 5' end in the region represented by the base sequence SEQ ID NO: 2;
the 5th to 10th CpG sites from the 5' end in the region represented by the base sequence SEQ ID NO: 3; and
the 13th to 27th CpG sites from the 5' end in the region represented by the base sequence SEQ ID NO: 4.

Methylation status can be analyzed by well-known methods that allow analysis on whether or not the CpG site to be analyzed is methylated. The well-known methods for analysis of methylation status may include, for example, bisulfite sequencing (see, e.g., T. Turan et al., "Methylation of human papillomavirus-18 L1 gene: A biomarker of neoplastic progression?" Virology 349 (2006) p.175-183), methylation specific PCR (see, James G.HERMAN et al., Methylation-specific PCR: A novel PCR assay for methylation status of CpG islands, Proc. Natl. Acad. Sci. USA, Vol.93, pp.9821-9826, September1996), a method of oxidizing methylated cytosine using a guide probe (see WO 2006/132022) and the like.

According to bisulfite sequencing, DNA methylation status can be analyzed as follows.

First, DNA extracted from a biological sample is subjected to reaction with a non-methylated cytosine conversion agent. The non-methylated cytosine conversion agent is a substance which converts non-methylated cytosine in DNA to a base other than cytosine (i.e. uracil, thymine, adenine or guanine). Such non-methylated cytosine conversion agent is preferably a bisulfite. The bisulfite which can be used may include sodium, potassium, calcium or magnesium bisulfite. DNA is treated with one or more of these bisulfites (bisulfite treatment). By the bisulfite treatment, non-methylated cytosine in DNA is converted to uracil, while methylated cytosine is not converted to uracil.

The concentration of the bisulfite during the bisulfite treatment is not specifically limited as long as it can sufficiently convert non-methylated cytosine(s) in DNA. The specific bisulfite concentration is usually 1 M or more, preferably 1 to 15 M and more preferably 3 to 10 M.

When sodium bisulfite is added to DNA extracted from a biological sample to the final concentration of 4 M in the mixture, non-methylated cytosine(s) can be converted to uracil(s) by incubating the mixture at 50 to 80°C for 10 to 90 min. When the bisulfite is used at a low concentration, time and temperature for the treatment may be appropriately varied in order to obtain sufficient conversion of non-methylated cytosine(s).

Next, the bisulfite-treated DNA is subjected to nucleic acid amplification using a primer set described below. After nucleic acid amplification, the obtained DNA fragment is sequenced for the base sequence of DNA after the bisulfite treatment. The methylation status of DNA can be analyzed from the determined base sequence.

Any well-known nucleic acid amplification methods such as PCR and LAMP can be used without limitation. The conditions for nucleic acid amplification can also be appropriately selected according to the type of the nucleic acid amplification method, the base sequence of the DNA fragment to be amplified, the base sequence of primers and the like.

The primer set which is used for bisulfite sequencing may be the one which can amplify DNA comprising a CpG site(s) to be analyzed for its(their) methylation status. Specifically, when the base sequence SEQ ID NO: 1 is to be analyzed, the following primer set can be used:
SEQ ID NO: 13: ttttatgagttatagatgtaggtgatagt; and
SEQ ID NO: 14: ccaccttaatcaccaaataacc.
When the base sequence SEQ ID NO: 2 is to be analyzed, the following primer set can be used:
SEQ ID NO: 15: taaaggatttgggattgagggtggg; and
SEQ ID NO: 16: ttcaaaaacatttctctaacaaaaaattc.
When the base sequence SEQ ID NO: 3 is to be analyzed, the following primer set can be used:
SEQ ID NO: 17: gggagttaggtttaggtggggatatg; and
SEQ ID NO: 18: aaaatcaaaaaacaaaaaacccttaac.
When the base sequence SEQ ID NO: 4 is to be analyzed, the following primer set can be used:
SEQ ID NO: 19: ttgtagtattattgttatagttttgtttttttt; and
SEQ ID NO: 20: attccactcctataataacatttatcaaaatctct.

According to methylation specific PCR (MSP), methylation status of DNA can be analyzed as follows.

First, non-methylated cytosine(s) in DNA extracted from a biological sample is converted to uracil by the bisulfite treatment. In this treatment, methylated cytosine(s) is not converted to uracil.

The bisulfite-treated DNA is subjected to nucleic acid amplification by PCR using a primer set described below. After the reaction, whether or not cytosine(s) of the CpG site(s) to be analyzed is(are) methylated can be determined according to the presence or absence of the nucleic acid amplified product.

The primer set which is used for methylation-specific PCR may be the one which can amplify a base sequence in which a cytosine(s) of a CpG site(s) to be analyzed has(have) not been converted to uracil, but can not amplify a base sequence in which cytosine(s) of the CpG site(s) has(have) been converted to uracil(s). When a nucleic acid amplified product is detected after the PCR reaction carried out with such primer set, it means that cytosine(s) of the CpG site(s) to be analyzed is(are) methylated.

Alternatively, a primer set can be used which can amplify a base sequence in which a cytosine(s) in a CpG site(s) to be analyzed has(have) been converted to uracil(s), but can not amplify a base sequence in which cytosine(s) in the CpG site(s) has(have) not been converted to uracil. When no nucleic acid amplified product is detected after the PCR reaction is carried out with such primer set, it means that cytosine(s) of the CpG site(s) to be analyzed is(are) methylated.

Primers contained in the primer set are preferably designed so as to include cytosine(s) of a CpG site(s) to be analyzed in the vicinity of the 3' end of the primers in order to improve specificity for methylated or non-methylated cytosine(s).

For example, when the CpG sites to be analyzed are the 3rd, 4th and 10th CpG sites from the 5' end of the base sequence SEQ ID NO: 1, the following primer set can be used:
SEQ ID NO: 5: gatgtaggtgatagttagcgatagcg; and
SEQ ID NO: 6:cctataaaaccgtctataaaaaaaacgaa.
When the CpG sites to be analyzed are the 28th and 30th CpG sites from the 5' end of the base sequence SEQ ID NO: 2, the following primer set can be used:
SEQ ID NO: 7: gaggagttgtgtggttttattgagtc; and
SEQ ID NO: 8: tctctaacaaaaaattccgaaacgta.
When the CpG site to be analyzed is the 6th CpG site from the 5' end of the base sequence SEQ ID NO: 3, the following primer set can be used:
SEQ ID NO: 9: ttagaggcgagtaagagttagggtagtc; and
SEQ ID NO: 10: acctacaaaaacgacacaaaaaacg.
When the CpG sites to be analyzed are the 23rd and 24th CpG sites from the 5' end of the base sequence SEQ ID NO: 4, the following primer set can be used:
SEQ ID NO: 11: gggttgttatttaaggttatattcgtacga;
SEQ ID NO: 12: taaaccgcaaatacgaaaacacgat.

In the present invention, the presence or absence of methylation of at least one CpG site may be analyzed by methods that can analyze whether or not a CpG site(s) to be analyzed is methylated, which may include, for example, bisulfite sequencing and methylation specific PCR as described above. Among them, methylation specific PCR is preferable because presence or absence of methylation can be determined conveniently and rapidly with a primer set specific to the CpG site(s) to be analyzed.

In the present invention, methylation frequency may be analyzed by methods that can analyze the ratio of the number of methylated CpG sites located in a region represented by a base sequence to be analyzed, which may include, for example, bisulfite sequencing and methylation-specific PCR as described above. Among them, bisulfite sequencing is preferable because it allows analysis of multiple CpG sites simultaneously in a region flanked by primers comprised in a primer set which has been designed so as to allow sequencing of the region represented by the base sequence to be analyzed.

As the total number of CpG sites located in a region represented by a base sequence to be analyzed is already known, the number of methylated CpG sites in the region itself can be regarded as a methylation frequency. The value obtained by dividing the number of methylated CpG sites in a region to be analyzed by the total number of CpG sites located in that region can also be regarded as a methylation frequency.

The presence or absence of a particular epithelial cancer-derived cell type in a biological sample can be determined based on the analysis result of the methylation status as described above.

For example, when an analysis result shows that at least one CpG site located in a region represented by the base sequences SEQ ID NO: 1, SEQ ID NO: 3 and SEQ ID NO: 4 is methylated, the decision that an epithelial cancer-derived cell is present can be given, while when an analysis result shows that the CpG site(s) is(are) not methylated, the decision that no epithelial cancer-derived cell is present can be given.

When an analysis result shows that at least one CpG site located in a region represented by the base sequence SEQ ID NO: 2 is not methylated, the decision that an epithelial cancer-derived cell is present can be given, while when an analysis result shows that the CpG site(s) is(are) methylated, the decision that no epithelial cancer-derived cell is present can be given.

Presence or absence of an epithelial cancer-derived cell can be determined based on a methylation frequency by comparing a methylation frequency of a region to be analyzed with a predetermined threshold.

More specifically, when a methylation frequency in a region represented by the base sequence SEQ ID NO: 1, SEQ ID NO: 3 or SEQ ID NO: 4 is higher than a threshold, the decision that an epithelial cancer-derived cell is present can be given, and when the methylation frequency is lower than the threshold, the decision that no epithelial cancer-derived cell is present can be given.

When a methylation frequency in a region represented by the base sequence SEQ ID NO: 2 is lower than a threshold, the decision that an epithelial cancer-derived cell is present can be given, and when the methylation frequency is higher than the threshold, the decision that no epithelial cancer-derived cell is present can be given.

The above threshold can be determined empirically. For example, it can be determined based on the results of methylation frequency analysis of a biological sample(s) which has been already confirmed to be free of any cancer cell and a biological sample(s) which has been already confirmed to contain an epithelial cancer-derived cell.

More specifically, when the value of the methylation frequency in an epithelial cancer-derived cell is higher than that in a normal cell, a threshold can be established which is higher than the methylation frequency in the normal cell and lower than the methylation frequency in the epithelial cancer-derived cell. When the value of the methylation frequency in an epithelial cancer-derived cell is lower than that in a normal cell, a threshold can be established which is lower than the methylation frequency in the normal cell and higher than the methylation frequency in the epithelial cancer-derived cell.

It is preferable to analyze the methylation frequency of more than one normal cell and the methylation frequency of more than one epithelial cancer-derived cell to establish a threshold that can differentiate epithelial cancer-derived cells from normal cells with the highest probability.

As described above, presence or absence of an epithelial cancer-derived cell can be determined by analyzing a CpG site(s) located in a region represented by the base sequences SEQ ID NOs: 1, 2, 3 and 4. Thus, a CpG site located in a region represented by the base sequences SEQ ID NOs: 1, 2, 3 and 4 can be a molecular marker for determination of the presence or absence of an epithelial cancer-derived cell using methylation analysis. A kit comprising a non-methylated cytosine conversion agent and a primer set which can be used in methylation-specific PCR can be used for determining the presence or absence of an epithelial cancer-derived cell in a biological sample.

The present invention is hereinafter illustrated in more detail by means of examples, which do not limit the present invention. Examples

### Example 1: Comprehensive methylation analysis by MeDIP-chip method using cell strains

Methylated DNA immunoprecipitation-microarray analysis (MeDIP-chip) was carried out to analyze the methylation status of breast cancer-derived cells.

### Preparation of test samples

### (1) Methylated DNA immunoprecipitation

Genomic DNA (4 µg) was extracted as biological samples from breast cancer-derived cell strains MCF7, MB-MDA231 and SKBR3 and a normal mammary epithelial cell strain HMEC and incubated overnight with the restriction enzyme *Mse*I (NEB) at 37°C to obtain fragments of 300 to 1000 bp. The biological samples after the reaction were denatured by heating them at 95°C for 10 min to obtain a single-stranded genomic DNA.

The denatured biological samples were diluted with a dilution buffer included in Chromatin Immunoprecipitation assay kit (Upstate biotechnology) according to the instruction attached to the kit and added with Protein G Sepharose beads (GE Healthcare). The mixture was rotated at 4°C for 30 min and centrifuged to collect the supernatant, thereby removing e.g. proteins that bind non-specifically to the beads. The collected supernatant was divided into two separate tubes, an anti-methylated cytosine antibody (for test samples) and normal mouse anti-IgG antibody (SantaCruz; for the control sample) were respectively added to the tubes and tubes were rotated overnight at 4°C.

Protein G Sepharose beads (GE Healthcare) were added to the tubes prior to rotation at 4°C for an hour, allowing the binding of the complex of the above antibody and the genomic DNA which was recognized by and bound to the antibodies to the beads, which were then recovered. The recovered beads were washed with a washing buffer included in the assay kit according to the instruction attached to the kit, and the genomic DNA was eluted from the immunoprecipitated complex using an elution buffer.

The genomic DNA obtained by the above methylated DNA immunoprecipitation method was subjected to the reaction with proteinase K prior to purification with Qiaquick PCR purification kit (QIAGEN) to obtain the test samples and the control sample.

### (2) Verification of test samples

PCR and agarose electrophoresis were carried out to verify if methylated DNA was specifically recovered by methylated DNA immunoprecipitation according to the above (1).

### (i) Preparation of PCR reaction solution

The following reagents were mixed to prepare a reaction solution of 25 µl.

| | |
|---|---|
| 2 x fastStart SYBR Green Master Mix (ROCHE) | 12.5 µl |
| Forward (F) primer (10 µM) | 1 µl |
| Reverse (Rv) primer (10 µM) | 1 µl |
| Genomic DNA (0.4 ng/µl) | 1 µl |
| dH₂O | 9.5 µl |

Base sequences of the primers used were as follows:

### <Primers for positive control>

"For cell strains MCF7 and HMEC: *GSTP1* primer sequences"
SEQ ID NO: 21: F: gaggccttcgctggagtt; and
SEQ ID NO: 22: Rv: gtactcactggtggcgaaga.

"For the cell strain MB-MDA231: *CDH1* primer sequences"
SEQ ID NO: 23: F: gtgaaccctcagccaatcag; and
SEQ ID NO: 24: Rv: agttccgacgccactgag.

"For the cell strain SKBR3: *ER* primer sequences"
SEQ ID NO: 25: F: gcctacgagttcaacgccg; and
SEQ ID NO: 26: Rv: aacgccgcagcctcagac.

### < Primers for negative controls (non-methylation probes) >

### "Primer sequences for non-methylated genes"

### (1) ch14-cgf1

SEQ ID NO: 27: F: ggaggagtcaagagaagttggaagc; and
SEQ ID NO: 28: Rv: cccacactccatttccattcctc.

### (2) ch14-cgf2

SEQ ID NO: 29: F: gggtactttgccaatatagccatgc; and
SEQ ID NO: 30: Rv: tggctaagtgggagggagaacag.

### (3) ch14-cgf3

SEQ ID NO: 31: F: ggatgggagacacctggttca and
SEQ ID NO: 32: Rv: ggatggaccagctgctttgtactc.

### (ii) PCR reaction conditions

PCR was carried out in the above reaction solution under the following conditions:
95°C for 10 min;
45 cycles of 95°C for 30 sec, 66°C for 15 sec and 72°C for 30 sec; and 1 cycle of 95°C for 1 min, 66°C for 30 sec and 95°C for 30 sec.

### (iii) Agarose electrophoresis

The above PCR products were subjected to electrophoresis on a 2% agarose gel to verify the amplified nucleic acids.

*GSTP1* gene has been known that its promoter region is methylated in the MCF7 cell strain but not in MB-MDA231 and HMEC cell strains. *ER* gene has been known that its promoter region is methylated in the SKBR3 cell strain but not in the MCF7 cell strain. *ch14-cgf1, ch14-cgf2* and *ch14-cgf3* genes have been known that they are not methylated.

### (3) Amplification and labeling of nucleic acids in test samples

Nucleic acids in the test and control samples were amplified using WT-Ovation™ Pico RNA Amplification System Version 1.0 (NuGEN) according to the instruction attached to the system. Absorbance (at 260 nm and 280 nm) of the samples was measured to determine the nucleic acid concentration.

The amplified nucleic acids in the test and control samples were fragmented and biotinylated with FL-Ovation™ cDNA Biotin Module V2 (NuGEN) according to the instruction attached to the system.

### Microarray analysis

### (1) Contact of samples with microarray

The prepared test and control samples were contacted with a microarray GeneChip^{®} Human Promoter 1.0R Array (Affymetrix) to be hybridized with probes on the microarray. Each one of the microarrays of the same type was used for the test and control samples, respectively. Staining, washing and scanning (measurement of signal) after the contact with samples were carried out by following the instruction provided by Affymetrix.

### (2) Analysis of methylation status

The array data obtained after scanning were analyzed. Array data analysis was carried out as follows: the Tiling Analysis Software (TAS; Affymetrix) was used to create a file (BAR file) of the regions in which the sample of the anti-methylated cytosine antibody had higher signal than the control, and then signals and p-values (-10log10(p-value)) from each probe were obtained. The regions having a statistically significant p-value below a certain threshold (such as -10log10(p-value) < 20) were created as BED files. The created BAR and BED files were analyzed with the default Integrated Genome Browser (Affymetrix).

Data containing annotation information corresponding to the p-values and signal values obtained from TAS was used to search for novel molecular markers that allow determination of presence or absence of breast cancer-derived cells.

### Results

Figs 1 to 4 show the base sequences of the regions identified by the analysis of the methylation status. According to the analysis, the regions SEQ ID NOs: 1, 3 and 4 were identified as DNA regions which were not methylated in the normal mammary epithelial cell strain but methylated in breast cancer cell strains. The region SEQ ID NO: 2 was identified as the DNA region which was methylated in the normal mammary epithelial cell strain but not methylated in breast cancer cell strains.

Thus, the regions SEQ ID NOs: 1 to 4 identified in Example 1 are the regions whose methylation status is different between normal cells and breast cancer-derived cells. Therefore, it suggests that these regions can be used as molecular markers for determination of presence or absence of epithelial cancer-derived cells by methylation analysis.

### Example 2: Analysis of methylation of CpG sites by bisulfite sequencing

The methylation status of the CpG sites included in the base sequences of the regions identified in Example 1 was analyzed by bisulfite sequencing. The methylation frequency and the methylation ratio of each CpG site were determined based on the analysis results.

### Bisulfite treatment

In order to carry out bisulfite sequencing, analysis samples were prepared by treating DNA extracted from cell lines and tissue-derived genomic DNA with bisulfite.

Human normal mammary tissue-derived genomic DNA was obtained by mixing three different lots of human normal mammary tissue-derived genomic DNA (BioChain). Breast cancer tissue-derived genomic DNA was obtained by mixing three different lots of human breast cancer tissue-derived genomic DNA (BioChain).

Each genomic DNA (2 µg) was added with 300 µl of 0.3 M NaOH and the mixture was incubated at 37°C for 10 min. For bisulfite treatment, 300 µl of a 10 M sodium bisulfite solution was added and the mixture was incubated at 80°C for 40 min. After the bisulfite treatment, DNA was purified from the solution using Qiaquick PCR purification kit (QIAGEN). Accordingly, the normal mammary tissue sample containing bisulfite-treated normal mammary tissue-derived genomic DNA and the breast cancer tissue sample containing bisulfite-treated breast cancer tissue-derived genomic DNA were obtained.

The genome was extracted from a breast cancer cell strain MCF7 and a normal mammary epithelial cell strain HMEC using QIAmp Blood Maxi kit (QIAGEN).

The extracted genomic DNA (2 µg) was added with 300 µl of 0.3 M NaOH and the mixture was incubated at 37°C for 10 min. For bisulfite treatment, 300 µl of a 10 M sodium bisulfite solution was added and the mixture was incubated at 80°C for 40 min. After the bisulfite treatment, DNA was purified from the solution using Qiaquick PCR purification kit (QIAGEN). Accordingly, the normal mammary cell strain sample containing bisulfite-treated normal mammary epithelial cell strain HMEC-derived genomic DNA and the breast cancer cell strain sample containing bisulfite-treated breast cancer cell strain MCF7-derived genomic DNA were obtained.

### PCR amplification of analysis samples

The purified normal mammary tissue sample, breast cancer tissue sample, normal mammary cell strain sample and breast cancer cell strain sample were subjected to PCR in the reaction solution described below to obtain PCR products.

Composition of the PCR reaction solution, reaction conditions and primers for the normal mammary tissue sample and the breast cancer tissue sample are as follows.

### <PCR reaction solution>

| | |
|---|---|
| 10 x Ex Taq Buffer (20 mM Mg2+plus) (TaKaRa) | 2.5 µL |
| dNTP Mixture (2.5 mM each) | 2 µL |
| F-primer (10 µM) | 1 µL |
| Rv-primer (10 µM) | 1 µL |
| Template | 1 µL |
| TaKaRa Ex Taq HS (5U/µl) | 0.2 µL |
| dH₂O | 17.3 µL |
| Total | 25 µL |

### <PCR conditions for amplification of DNA in the normal mammary tissue sample and the breast cancer tissue sample>

95°C for 4.5 min; and
40 cycles of 95°C for 30 sec, 57.3°C for 30 sec and 72°C for 30sec.

### <Primer sequences for sequencing the region SEQ ID NO: 1>

SEQ ID NO: 13: F: ttttatgagttatagatgtaggtgatagt; and
SEQ ID NO: 14: Rv: ccaccttaatcaccaaataacc

### <Primer sequences for sequencing the region SEQ ID NO: 2>

SEQ ID NO: 15: F: taaaggatttgggattgagggtggg; and
SEQ ID NO: 16: Rv: ttcaaaaacatttctctaacaaaaaattc

### <Primer sequences for sequencing the region SEQ ID NO: 4>

SEQ ID NO: 19: F: ttgtagtattattgttatagttttgtttttttt; and
SEQ ID NO: 20: Rv: attccactcctataataacatttatcaaaatctct

PCR reaction solution, reaction conditions and primers for the normal mammary cell line sample and the breast cancer cell strain sample are as follows.

### <PCR reaction solution>

| | |
|---|---|
| 10 x Ex Taq Buffer (20 mM Mg2+plus) (TaKaRa) | 2.5 µL |
| dNTP Mixture (2.5 mM each) | 2 µL |
| F-primer (10 µM) | 1 µL |
| Rv-primer (10 µM) | 1 µL |
| Template | 1 µL |
| TaKaRa Ex Taq HS (5U/µl) | 0.2 µL |
| dH₂O | 17.3 µL |
| Total | 25 µL |

### <PCR conditions for amplification of DNA in the normal mammary cell strain sample and the breast cancer cell strain sample>

95°C for 4.5 min; and
40 cycles of 95°C for 30 sec, 57.6°C for 30 sec and 72°C for 30sec.

### <Primer sequences for sequencing the region SEQ ID NO: 3>

SEQ ID NO: 17: F: gggagttaggtttaggtggggatatg; and
SEQ ID NO: 18: Rv: aaaatcaaaaaacaaaaaacccttaac

### <Primer sequences for sequencing the region SEQ ID NO: 4>

SEQ ID NO: 19: F: ttgtagtattattgttatagttttgtttttttt; and
SEQ ID NO: 20: Rv: attccactcctataataacatttatcaaaatctct

### Cloning of PCR products

The amplified products from the above PCR were incorporated into a vector using a TA cloning kit (TOPO TA Cloning kit (Invitrogen)). The constructs in the vectors were used for transformation of *Escherichia coli* (TOP10). The transformed *E*. *coli* was incubated overnight in an LB agar medium (composition: 1% (w/v) Tryptone, 0.5% (w/v) yeast extract, 1% (w/v) sodium chloride and 1.5% (w/v) agar) at 37°C.

### Sequencing

Plasmid was purified from *E*. *coli* obtained after incubation using GenElute Plasmid Miniprep kit (SIGMA). The base sequence of the amplified product in the purified plasmid was determined by BigDye terminator Cycle Sequencing using a gene analysis system (Applied Biosystems 3730x1 DNA Analyzer (Applied Biosystems)).

After the sequencing, the samples were analyzed for whether or not CpG sites were methylated.

### Results

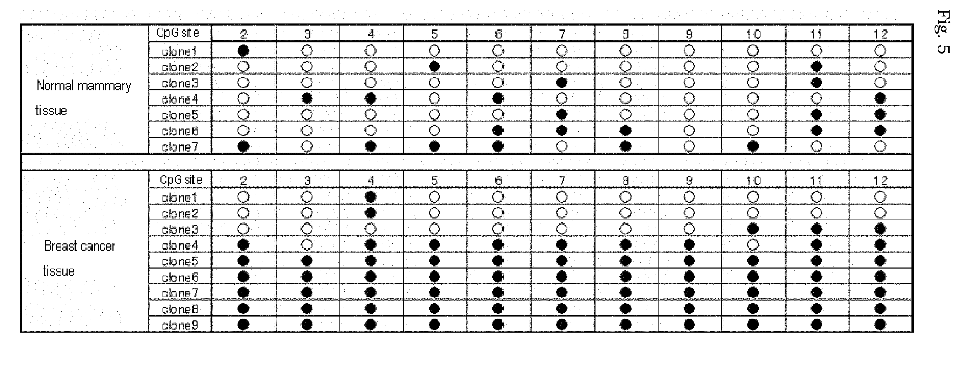

Analysis results on methylation in the regions (SEQ ID NOs: 1 to 4) are shown in Figs. 5 to 9. Methylated CpG sites are marked with "filled circle" and non-methylated CpG sites are marked with "open circle".

Fig. 5 shows presence or absence of methylation in the 2nd to 12th CpG sites from the 5' end of the base sequence SEQ ID NO: 1 in seven normal mammary tissue samples and nine breast cancer tissue samples.

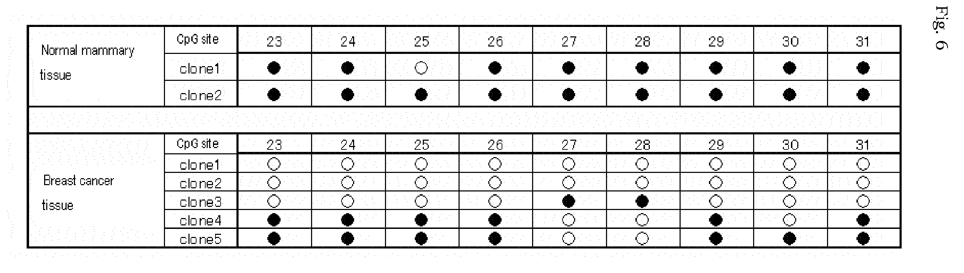

Fig. 6 shows presence or absence of methylation in the 23rd to 31st CpG sites from the 5' end of the base sequence SEQ ID NO: 2 in two normal mammary tissue samples and five breast cancer tissue samples.

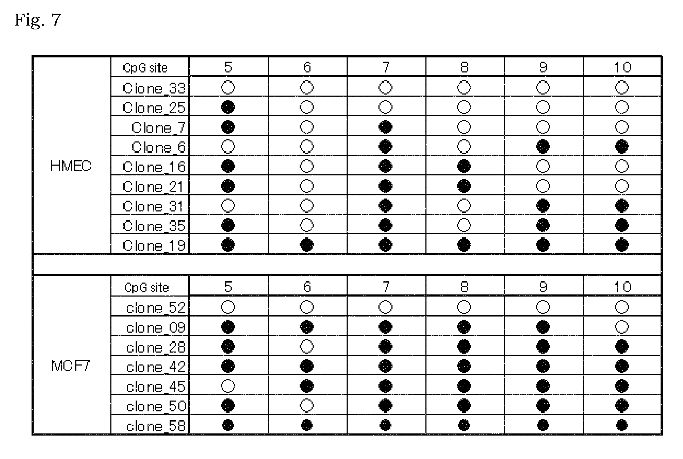

Fig. 7 shows presence or absence of methylation in the 5th to 10th CpG sites from the 5' end of the base sequence SEQ ID NO: 3 in nine normal mammary epithelial cell strain (HMEC) samples and seven breast cancer-derived cell strain (MCF7) samples.

Fig. 8 shows presence or absence of methylation in the 13th to 24th CpG sites from the 5' end of the base sequence SEQ ID NO: 4 in seven normal mammary epithelial cell strain (HMEC) samples and seven breast cancer-derived cell strain (MCF7) samples.

Fig. 9 shows presence or absence of methylation in the 13th to 27th CpG sites from the 5' end of the base sequence SEQ ID NO: 4 in four normal mammary tissue samples and four breast cancer tissue samples.

Graphs in Figs. 10 to 14 show the methylation frequency of the regions analyzed based on the results of Figs. 5 to 9.

The threshold was established as 50% based on the methylation frequency in normal cells (normal mammary tissue or normal mammary epithelial cell strain) and the methylation frequency in breast cancer-derived cells (breast cancer tissue or breast cancer-derived cell strain).

The methylation frequency of the regions represented by the base sequences SEQ ID NOs: 1, 3 and 4 is lower than the threshold in normal cells and higher than the determined threshold in breast cancer-derived cells.

This suggests that the analysis results showing higher methylation frequency of the regions SEQ ID NOs: 1, 3 and 4 included in DNA extracted from biological samples obtained from test subjects can provide the determination that epithelial cancer-derived cells are present in the biological samples.

The methylation frequency of the region represented by the base sequence SEQ ID NO: 2 is higher than the threshold in normal cells and lower than the determined threshold in breast cancer-derived cells.

This suggests that the analysis results showing lower methylation frequency of the region SEQ ID NO: 2 included in DNA extracted from biological samples obtained from test subjects can provide the determination that epithelial cancer-derived cells are present in the biological samples.

Tables in Figs. 15 to 19 show the ratio of cells whose respective CpG sites are methylated, based on the results of Figs. 5 to 9.

Figs. 15 to 19 show that the methylation ratio of CpG sites located in base sequences SEQ ID NOs: 1, 3 and 4 is higher in breast cancer-derived cells and the methylation ratio of CpG sites located in the base sequence SEQ ID NO: 2 is higher in normal cells.

This suggests that the analysis results showing methylation of CpG sites located in base sequences SEQ ID NOs: 1, 3 and 4 can provide the determination that breast cancer-derived cells are present in biological samples and the analysis results showing no methylation of CpG sites located in the base sequence SEQ ID NO: 2 can provide the determination that breast cancer-derived cells are present in biological samples.

Among them, the CpG sites having a large variation in the methylation ratio between normal cells and breast cancer-derived cells are suitable for analysis of the methylation status. The primers for methylation specific PCR designed to target such CpG sites may provide more precise determination on presence or absence of breast cancer-derived cells.

### Example 3: Detection of breast cancer-derived cells by methylation specific PCR

The methylation status of CpG sites in the base sequences SEQ ID NOs: 1, 3 and 4 in breast cancer genomic DNA was analyzed by methylation specific DNA.

Normal human mammary tissue-derived genomic DNA (BioChain) of three different lots and human breast cancer tissue-derived genomic DNA (BioChain) of three different lots were used as genomic DNAs.

The genomic DNA (2 µg) was added with 300 µl of 0.3 M NaOH and the mixture was incubated at 37°C for 10 min. For bisulfite treatment, 300 µl of a 10 M sodium bisulfite solution was added and the mixture was incubated at 80°C for 40 min. After the bisulfite treatment, DNA was purified from the solution using Qiaquick PCR purification kit (QIAGEN). Accordingly, the normal mammary tissue genomic samples A, B and C containing bisulfite-treated normal mammary tissue-derived genomic DNA and the breast cancer tissue samples D, E and F containing bisulfite-treated breast cancer tissue-derived genomic DNA were obtained.

PCR reactions for methylation analysis of SEQ ID NO: 1 and SEQ ID NO: 4 were carried out with the following reaction solution, reaction conditions and primers.

The methylation specific PCR primers for SEQ ID NO: 1 is to analyze methylation of the targets, i.e. the 3rd, 4th and 10th CpGs from the 5' end of the base sequence SEQ ID NO: 1. The methylation specific PCR primers for SEQ ID NO: 4 is to analyze methylation of the targets, i.e. the 23rd and 24th CpGs from the 5' end of the base sequence SEQ ID NO: 4.

### <PCR reaction solution>

| | |
|---|---|
| 2 x FastStart SYBR Green Master Mix (ROCHE) | 12.5 µL |
| F-primer (10 µM) | 1 µL |
| Rv-primer (10 µM) | 1 µL |
| Template | 1 µL |
| dH₂O | 9.5 µL |
| Total | 25 µL |

### <PCR reaction conditions for SEQ ID NOs: 1 and 4>

95°C for 10 min;
33 cycles of 95°C for 30 sec, 62°C for 30 sec and 72°C for 30 sec; and 1 cycle of 95°C for 1 min, 62°C for 30 sec and 95°C for 30 sec.

### <Methylation specific PCR primers for SEQ ID NO: 1>

SEQ ID NO: 5: F: gatgtaggtgatagttagcgatagcg; and
SEQ ID NO: 6: Rv: cctataaaaccgtctataaaaaaaacgaa

### <Methylation specific PCR primers for SEQ ID NO: 4>

SEQ ID NO: 11: F: gggttgttatttaaggttatattcgtacga; and
SEQ ID NO: 12: Rv: taaaccgcaaatacgaaaacacgat

PCR reaction for SEQ ID NO: 3 was carried out with the following reaction solution, reaction conditions and primers. The methylation specific PCR primers for SEQ ID NO: 3 are to analyze methylation of the target, i.e. the 6th CpG from the 5' end of the base sequence SEQ ID NO: 3.

### <PCR reaction solution>

| | |
|---|---|
| 2 x FastStart SYBR Green Master Mix (ROCHE) | 12.5 µL |
| F-primer (10 µM) | 1 µL |
| Rv-primer (10 µM) | 1 µL |
| Template | 1 µL |
| dH₂O | 9.5 µL |
| Total | 25 µL |

### <PCR reaction conditions for SEQ ID NO: 3>

95°C for 10 min;
35 cycles of 95°C for 30 sec, 62°C for 30 sec and 72°C for 30 sec; and
1 cycle of 95°C for 1 min, 62°C for 30 sec and 95°C for 30 sec.

### <Methylation specific PCR primers for SEQ ID NO: 3>

SEQ ID NO: 9: F: ttagaggcgagtaagagttagggtagtc; and
SEQ ID NO: 10: Rv: acctacaaaaacgacacaaaaaacg

The amplified products from methylation specific PCR were visualized by agarose gel electrophoresis.

### Results

Figs. 17 to 19 show the results of agarose gel electrophoresis of the amplified products from methylation specific PCR.

It was confirmed that in any of Fig. 17 (methylation specific PCR analysis of SEQ ID NO: 1), Fig. 18 (methylation specific PCR analysis of SEQ ID NO: 3) and Fig. 19 (methylation specific PCR analysis of SEQ ID NO: 4), the amount of amplified products in the breast cancer tissue samples D, E, and F is higher than that in the normal mammary tissue samples A, B and C.

This suggests that methylation specific PCR analysis of CpG sites located in the base sequences SEQ ID NOs: 1 to 4 included in DNA extracted from biological samples obtained from test subjects can provide determination on presence or absence of breast cancer-derived cells in the biological samples.

### Example 4: Methylation specific PCR analysis of CpG sites included in PCDHGA10 gene (SEQ ID NO: 1) in epithelial cancer-derived cells

The methylation status of CpG sites included in the base sequence of SEQ ID NO: 1 in genomic DNAs of large bowel cancer, gastric cancer and cervical cancer cells were analyzed by methylation specific PCR.

QIAmp Blood Maxi kit (QIAGEN) was used to extract the genome from a large bowel cancer cell strain HCT16, a gastric cancer cell strain KATO3 and cervical cancer cell strains C33A and SiHa. The extracted genomic DNA (2 µg) was added with 300 µl of 0.3 M NaOH and the mixture was incubated at 37°C for 10 min. For bisulfite treatment, 300 µl of a 10 M sodium bisulfite solution was added and the mixture was incubated at 80°C for 40 min. After the bisulfite treatment, DNA was purified from the solution using Qiaquick PCR purification kit (QIAGEN). Accordingly, the large bowel cancer HCT16 sample containing bisulfite-treated HCT16-derived genomic DNA, the gastric cancer KATO3 sample containing bisulfite-treated KATO3-derived genomic DNA, the cervical cancer C33A sample containing bisulfite-treated C33A-derived genomic DNA and the cervical cancer SiHa sample containing bisulfite-treated SiHa-derived genomic DNA were obtained.

Human large bowel cancer tissue-derived genomic DNA (BioChain) of two different lots and human normal large bowel tissue-derived genomic DNA (BioChain) of one lot were treated in the similar manner as the cell strains described above. Accordingly, two large bowel cancer tissue samples (large bowel cancer tissue samples A and B) containing bisulfite-treated large bowel cancer tissue-derived genomic DNA and the normal large bowel tissue sample containing bisulfite-treated normal large bowel tissue-derived genomic DNA were obtained.

The obtained samples were subjected to methylation specific PCR in order to analyze methylation of CpG sites included in the base sequence SEQ ID NO: 1.

PCR reaction for methylation analysis of SEQ ID NO: 1 was carried out with the reaction solution, reaction conditions and primers described in Example 3.

The amplified products of methylation specific PCR were visualized by agarose gel electrophoresis.

### Results

Fig. 23 shows the results of agarose gel electrophoresis of the amplified products obtained from methylation specific PCR of SEQ ID NO: 1. This result shows that the amount of the amplified product in the large bowel cancer tissue samples A and B is higher than that in the normal large bowel tissue sample. It was also found that almost the same amount of the amplified product was confirmed for the large bowel cancer HCT16 sample, the gastric cancer KATO3 sample, the cervical cancer C33A sample and the cervical cancer SiHa sample as the large bowel cancer tissue samples A and B.

These results suggest that methylation analysis of the CpG sites located in the base sequence SEQ ID NO: 1 by methylation specific PCR can provide determination on presence or absence of epithelial cancer-derived cells such as breast cancer-, large bowel cancer-, gastric cancer- and cervical cancer-derived cells.

### Example 5: Methylation specific PCR analysis of CpG sites included in PCDHB6 gene (SEQ ID NO: 2) in epithelial cancer-derived cells

The methylation status of CpG sites included in the base sequence of SEQ ID NO: 2 in genomic DNA of large bowel cancer cells were analyzed by methylation specific PCR.

Two large bowel cancer tissue samples (large bowel cancer tissue samples A and B) and the normal large bowel tissue sample obtained in Example 4 were used as analysis samples.

PCR reaction for methylation analysis of the base sequence SEQ ID NO: 2 was carried out with the following reaction solution, reaction conditions and primers.

The methylation specific PCR primers for SEQ ID NO: 2 are to analyze methylation of the targets, i.e. the 28th and 30th CpGs from the 5' end of the base sequence SEQ ID NO: 2.

### <PCR reaction solution>

| | |
|---|---|
| 2 x FastStart SYBR Green Master Mix (ROCHE) | 12.5 µL |
| F-primer (10 µM) | 1 µL |
| Rv-primer (10 µM) | 1 µL |
| Template | 1 µL |
| dH₂O | 9.5 µL |
| Total | 25 µL |

### <PCR reaction conditions for SEQ ID NO: 2>

95°C for 10 min;
33 cycles of 95°C for 30 sec, 62°C for 30 sec and 72°C for 30 sec; and
1 cycle of 95°C for 1 min, 62°C for 30 sec and 95°C for 30 sec.

### <Methylation specific PCR primers for SEQ ID NO: 2>

SEQ ID NO: 7: F: gaggagttgtgtggttttattgagtc; and
SEQ ID NO: 8: Rv: tctctaacaaaaaattccgaaacgta

The amplified products from methylation specific PCR were visualized by agarose electrophoresis.

### Results

Fig. 24 shows the results of agarose gel electrophoresis of the amplified products from methylation specific PCR of *PCDHB6* gene (SEQ ID NO: 2). From this result, it was confirmed that the amount of the amplified product in the normal large bowel tissue sample was higher than that in the large bowel cancer tissue samples A and B.

This suggests that methylation analysis of the CpG sites located in the base sequence SEQ ID NO: 2 by methylation specific PCR can provide determination on presence or absence of epithelial cancer-derived cells such as large bowel cancer-derived cells.

### Example 6: Methylation specific PCR analysis of CpG sites included in LBX2 gene (SEQ ID NO: 3) in epithelial cancer-derived cells

The methylation status of CpG sites included in the base sequence SEQ ID NO: 3 in genomic DNAs of large bowel cancer and cervical cancer cells were analyzed by methylation specific PCR.

The large bowel cancer HCT16 sample, the cervical cancer C33A sample and the cervical cancer SiHa sample, as well as two large bowel cancer tissue samples (large bowel cancer tissue samples A and B) and the normal large bowel tissue sample obtained in Example 4 were used as analysis samples.

PCR reaction for methylation analysis of the base sequence SEQ ID NO: 3 was carried out with the reaction solution, reaction conditions and primers described in Example 3.

The amplified products from methylation specific PCR were visualized by agarose electrophoresis.

### Results

Fig. 25 shows the results of agarose gel electrophoresis of the amplified products by methylation specific PCR of the base sequence SEQ ID NO: 3. From this result, it was confirmed that the amount of the amplified product in the large bowel cancer tissue samples A and B was higher than that in the normal large bowel tissue sample. It was also found that almost the same amount of the amplified product was confirmed for the large bowel cancer HCT16 sample, the cervical cancer C33A sample and the cervical cancer SiHa sample as the large bowel cancer tissue samples A and B.

These results suggest that methylation analysis of the CpG sites located in the base sequence SEQ ID NO: 3 by methylation specific PCR can provide determination on presence or absence of epithelial cancer-derived cells such as breast cancer-, large bowel cancer- and cervical cancer-derived cells.

### Example 7: Methylation specific PCR analysis of CpG sites included in the base sequence SEQ ID NO: 4 in epithelial cancer-derived cells

The methylation status of CpG sites included in the chromosome 1 gene (SEQ ID NO: 4) in genomic DNAs of large bowel cancer, gastric cancer and cervical cancer cells were analyzed by methylation specific PCR.

The large bowel cancer HCT16 sample, the gastric cancer KATO3 sample, the cervical cancer C33A sample and the cervical cancer SiHa sample, as well as two large bowel cancer tissue samples (large bowel cancer tissue samples A and B) and the normal large bowel tissue sample were used as analysis samples.

PCR reaction for methylation analysis of the base sequence SEQ ID NO: 4 was carried out with the reaction solution, reaction conditions and primers described in Example 3.

The amplified products from methylation specific PCR were visualized by agarose gel electrophoresis.

### Results

Fig. 26 shows the results of agarose gel electrophoresis of the amplified products from methylation specific PCR of the base sequence SEQ ID NO: 4. From this result, it was confirmed that the amount of the amplified product in the large bowel cancer tissue samples A and B was higher than that in the normal large bowel tissue sample. It was also found that almost the same amount of the amplified product was confirmed for the large bowel cancer HCT16 sample, the gastric cancer KATO3 sample, the cervical cancer C33A sample and the cervical cancer SiHa sample as the large bowel cancer tissue samples A and B.

These results suggest that methylation analysis of the CpG sites located in the base sequence SEQ ID NO: 4 by methylation specific PCR can provide determination on presence or absence of epithelial cancer-derived cells such as breast cancer-, large bowel cancer-, gastric cancer- and cervical cancer-derived cells.

## Claims

1. A method for determining the presence or absence of a breast cancer-derived cell, large bowel cancer-derived cell, gastric cancer-derived cell or cervical cancer-derived cell in a biological sample obtained from a subject comprising the steps of:
extracting DNA from the biological sample,
analyzing the methylation status of a CpG site located in the region represented by base sequence SEQ ID NO: 1 in the DNA obtained from the step of extracting, wherein when the presence or absence of the breast cancer-derived cell is determined, the CpG site to be analyzed for its methylation status is at least one selected from the group consisting of the 2^{nd} to 12^{th} CpG sites from the 5'end in the region represented by base sequence SEQ ID NO:1, and
determining the presence or absence of the breast cancer-derived cell, large bowel cancer-derived cell, gastric cancer-derived cell or cervical cancer-derived cell in the biological sample based on the analysis result obtained from the step of analyzing.

2. The method according to claim 1, wherein in the step of analyzing, the presence or absence of methylation of at least one CpG site located in the region represented by base sequence SEQ ID NO: 1 is determined.

3. The method according to claim 2, wherein in the step of determining, it is determined that the large bowel cancer-derived cell, gastric cancer-derived cell or cervical cancer-derived cell is present in the biological sample when the analysis result obtained from the step of analyzing shows methylation of the CpG site located in the region represented by base sequence SEQ ID NO: 1.

4. The method according to claim 2, wherein in the step of determining, it is determined that the breast cancer-derived cell is present in the biological sample when the analysis result obtained from the step of analyzing shows methylation of the at least one CpG site selected from the group consisting of the 2^{nd} to 12^{th} CpG sites from the 5'end in the region represented by base sequence SEQ ID NO:1.

5. The method according to claim 1, wherein in the step of analyzing, a methylation frequency of the CpG site located in the region represented by the base sequence SEQ ID NO: 1 is analyzed.

6. The method according to claim 5, wherein in the step of determining, it is determined that the large bowel cancer-derived cell, gastric cancer-derived cell or cervical cancer-derived cell is present in the biological sample when the analysis result obtained from the step of analyzing shows high methylation frequency in the CpG site located in at least one region represented by base sequence SEQ ID NO: 1.

7. The method according to claim 5, wherein in the step of determining, it is determined that the breast cancer-derived cell is present in the biological sample when the analysis result obtained from the step of analyzing shows high methylation frequency in the at least one CpG site selected from the group consisting of the 2^{nd} to 12^{th} CpG sites from the 5'end in the region represented by base sequence SEQ ID NO:1.

8. The method according to claim 1, wherein the CpG site is selected from:
the 2nd to 12th CpG sites from the 5' end in the region represented by the base sequence SEQ ID NO: 1.

9. The method according to any of claims 1 to 8, wherein the CpG site is selected from the 3^{rd}, the 4^{th} and the 10^{th} CpG sites from the 5' end in the region represented by the base sequence SEQ ID NO:1.

10. A kit for determining the presence or absence of a breast cancer-derived cell, large bowel cancer-derived cell, gastric cancer-derived cell or cervical cancer-derived cell in a biological sample obtained from a subject comprising:
a non-methylated cytosine conversion agent that converts non-methylated cytosine in DNA extracted from the biological sample to a different base; and
a primer set for detection of methylation of at least one CpG site located in a region represented by a base sequence SEQ ID NO: 1 by methylation specific PCR, wherein said primer set is selected from primers represented by base sequences SEQ ID NOs: 5 and 6.

11. The kit according to claim 10, wherein the CpG site is selected from: the 3^{rd} , the 4^{th} and the 10^{th} CpG sites from the 5' end in the region represented by the base sequence SEQ ID NO: 1.

## Patentansprüche

1. Verfahren zur Bestimmung des Vorliegens oder Fehlens einer aus Brustkrebs, Dickdarmkrebs, Magenkrebs oder Gebärmutterhalskrebs stammenden Zelle in einer von einem Individuum erhaltenen biologischen Probe, umfassend die Schritte:
Extrahieren von DNA aus der biologischen Probe,
Analysieren des Methylierungsstatus einer in dem durch die Basensequenz SEQ ID NO: 1 repräsentierten Bereich in der aus dem Extraktionsschritt erhaltenen DNA lokalisierten CpG-Stelle, wobei, wenn das Vorliegen oder Fehlen der aus Brustkrebs stammenden Zelle bestimmt wird, es sich bei der auf ihren Methylierungsstatus zu analysierenden CpG-Stelle um wenigstens eine aus der aus der 2. bis 12. CpG-Stelle vom 5'-Ende im durch die Basensequenz SEQ ID NO: 1 repräsentierten Bereich bestehenden Gruppe ausgewählte Stelle handelt, und
Bestimmen des Vorliegens oder Fehlens der aus Brustkrebs, Dickdarmkrebs, Magenkrebs oder Gebärmutterhalskrebs stammenden Zelle in der biologischen Probe bezogen auf das aus dem Analysierschritt erhaltene Analyseergebnis.

2. Verfahren nach Anspruch 1, wobei im Analysierschritt das Vorliegen oder Fehlen einer Methylierung wenigstens einer in dem durch die Basensequenz SEQ ID NO: 1 repräsentierten Bereich lokalisierten CpG-Stelle bestimmt wird.

3. Verfahren nach Anspruch 2, wobei im Bestimmungsschritt bestimmt wird, dass die aus Dickdarmkrebs, Magenkrebs oder Gebärmutterhalskrebs stammende Zelle in der biologischen Probe vorliegt, wenn das aus dem Analysierschritt erhaltene Analyseergebnis eine Methylierung der in dem durch die Basensequenz SEQ ID NO: 1 repräsentierten Bereich lokalisierten CpG-Stelle zeigt.

4. Verfahren nach Anspruch 2, wobei im Bestimmungsschritt bestimmt wird, dass die aus Brustkrebs stammende Zelle in der biologischen Probe vorliegt, wenn das aus dem Analysierschritt erhaltene Analyseergebnis eine Methylierung der wenigstens einen aus der aus der 2. bis 12. CpG-Stelle vom 5'-Ende im durch die Basensequenz SEQ ID NO: 1 repräsentierten Bereich bestehenden Gruppe ausgewählten CpG-Stelle zeigt.

5. Verfahren nach Anspruch 1, wobei im Analysierschritt eine Methylierungsfrequenz der in dem durch die Basensequenz SEQ ID NO: 1 repräsentierten Bereich lokalisierten CpG-Stelle analysiert wird.

6. Verfahren nach Anspruch 5, wobei im Bestimmungsschritt bestimmt wird, dass die aus Dickdarmkrebs, Magenkrebs oder Gebärmutterhalskrebs stammende Zelle in der biologischen Probe vorliegt, wenn das aus dem Analysierschritt erhaltene Analyseergebnis eine hohe Methylierungsfrequenz an der in wenigstens einem durch die Basensequenz SEQ ID NO: 1 repräsentierten Bereich lokalisierten CpG-Stelle zeigt.

7. Verfahren nach Anspruch 5, wobei im Bestimmungsschritt bestimmt wird, dass die aus Brustkrebs stammende Zelle in der biologischen Probe vorliegt, wenn das aus dem Analysierschritt erhaltene Analyseergebnis eine hohe Methylierungsfrequenz an der wenigstens einen aus der aus der 2. bis 12. CpG-Stelle vom 5'-Ende im durch die Basensequenz SEQ ID NO: 1 repräsentierten Bereich bestehenden Gruppe ausgewählten CpG-Stelle zeigt.

8. Verfahren nach Anspruch 1, wobei die CpG-Stelle ausgewählt ist aus:
der 2. bis 12. CpG-Stelle vom 5 '-Ende im durch die Basensequenz SEQ ID NO: 1 repräsentierten Bereich.

9. Verfahren nach einem der Ansprüche 1 bis 8, wobei die CpG-Stelle aus der 3., der 4. und der 10. CpG-Stelle vom 5'-Ende im durch die Basensequenz SEQ ID NO: 1 repräsentierten Bereich ausgewählt ist.

10. Kit zur Bestimmung des Vorliegens oder Fehlens einer aus Brustkrebs, Dickdarmkrebs, Magenkrebs oder Gebärmutterhalskrebs stammenden Zelle in einer von einem Individuum erhaltenen biologischen Probe, umfassend:
ein Nichtmethyliertes-Cytosin-Umwandlungsmittel, das nichtmethyliertes Cytosin in aus der biologischen Probe extrahierter DNA in eine andere Base überführt; und
einen Primer-Satz zum Nachweis einer Methylierung wenigstens einer in einem durch eine Basensequenz SEQ ID NO: 1 repräsentierten Bereich lokalisierten CpG-Stelle mittels methylierungsspezifischer PCR, wobei der Primer-Satz aus durch die Basensequenzen SEQ ID NO: 5 und 6 repräsentierten Primern ausgewählt ist.

11. Kit nach Anspruch 10, wobei die CpG-Stelle aus der 3., der 4. und der 10. CpG-Stelle vom 5'-Ende im durch die Basensequenz SEQ ID NO: 1 repräsentierten Bereich ausgewählt ist.

## Revendications

1. Procédé pour déterminer la présence ou l'absence d'une cellule dérivée de cancer du sein, cellule dérivée de cancer du gros intestin, cellule dérivée de cancer de l'estomac ou cellule dérivée de cancer de l'estomac dans un échantillon biologique obtenu à partir d'un sujet comprenant les étapes de :
extraction d'ADN à partir de l'échantillon biologique,
analyse du statut de méthylation d'un site CpG situé dans la région représentée par la séquence de bases SEQ ID NO: 1 dans l'ADN obtenu à partir de l'étape d'extraction, dans lequel, lorsque la présence ou l'absence de la cellule dérivée de cancer du sein est déterminée, le site CpG à analyser pour son statut de méthylation est au moins l'un choisi dans le groupe constitué des 2^{ème} à 12^{ème} sites CpG à partir de l'extrémité 5' dans la région représentée par la séquence de bases SEQ ID NO: 1, et
détermination de la présence ou l'absence de la cellule dérivée de cancer du sein, cellule dérivée de cancer du gros intestin, cellule dérivée de cancer de l'estomac ou cellule dérivée de cancer de l'estomac dans l'échantillon biologique sur la base du résultat d'analyse obtenu à partir de l'étape d'analyse.

2. Procédé selon la revendication 1, dans lequel, dans l'étape d'analyse, la présence ou l'absence de méthylation d'au moins un site CpG situé dans la région représentée par la séquence de bases SEQ ID NO: 1 est déterminée.

3. Procédé selon la revendication 2, dans lequel, dans l'étape de détermination, il est déterminé que la cellule dérivée de cancer du gros intestin, cellule dérivée de cancer de l'estomac ou cellule dérivée de cancer de l'estomac est présente dans l'échantillon biologique lorsque le résultat d'analyse obtenu à partir de l'étape d'analyse montre que la méthylation du site CpG situé dans la région représentée par la séquence de bases SEQ ID NO: 1.

4. Procédé selon la revendication 2, dans lequel, dans l'étape de détermination, il est déterminé que la cellule dérivée de cancer du sein est présente dans l'échantillon biologique lorsque le résultat d'analyse obtenu à partir de l'étape d'analyse indique la méthylation de l'au moins un site CpG choisi dans le groupe constitué des 2^{ème} à 12^{ème} sites CpG à partir de l'extrémité 5' dans la région représentée par la séquence de bases SEQ ID NO: 1.

5. Procédé selon la revendication 1, dans lequel, dans l'étape d'analyse, une fréquence de méthylation du site CpG situé dans la région représentée par la séquence de bases SEQ ID NO: 1 est analysé.

6. Procédé selon la revendication 5, dans lequel, dans l'étape de détermination, il est déterminé que la cellule dérivée de cancer du gros intestin, cellule dérivée de cancer de l'estomac ou cellule dérivée de cancer de l'estomac est présente dans l'échantillon biologique lorsque le résultat d'analyse obtenu à partir de l'étape d'analyse indique une fréquence de méthylation élevée dans le site CpG situé dans au moins une région représentée par la séquence de bases SEQ ID NO: 1.

7. Procédé selon la revendication 5, dans lequel, dans l'étape de détermination, il est déterminé que la cellule dérivée de cancer du sein est présente dans l'échantillon biologique lorsque le résultat d'analyse obtenu à partir de l'étape d'analyse indique une fréquence de méthylation élevée dans l'au moins un site CpG choisi dans le groupe constitué des 2^{ème} à 12^{ème} sites CpG de l'extrémité 5' dans la région représentée par la séquence de bases SEQ ID NO: 1.

8. Procédé selon la revendication 1, dans lequel le site CpG est choisi parmi :
les 2^{ème} à 12^{ème} sites CpG à partir de l'extrémité 5' dans la région représentée par la séquence de bases SEQ ID NO: 1.

9. Procédé selon l'une quelconque des revendications 1 à 8, dans lequel le site CpG est choisi parmi le 3^{ème}, le 4^{ème} et le 10^{ème} sites CpG à partir de l'extrémité 5' dans la région représentée par la séquence de bases SEQ ID NO: 1.

10. Kit pour déterminer la présence ou l'absence d'une cellule dérivée de cancer du sein, cellule dérivée de cancer du gros intestin, cellule dérivée de cancer de l'estomac ou cellule dérivée de cancer de l'estomac dans un échantillon biologique obtenu à partir d'un sujet comprenant :
un agent de conversion de cytosine non méthylée qui convertit la cytosine non méthylée dans l'ADN extrait à partir de l'échantillon biologique en une base différente ; et
un ensemble d'amorces défini pour la détection de la méthylation d'au moins un site CpG situé dans une région représentée par une séquence de bases SEQ ID NO: 1 par PCR méthylation-spécifique, où ledit ensemble d'amorces est choisi parmi des amorces représentées par des séquences de base SEQ ID NO: 5 et 6.

11. Kit selon la revendication 10, dans lequel le site CpG est choisi parmi : le 3^{ème}, le 4^{ème} et le 10^{ème} sites CpG à partir de l'extrémité 5' dans la région représentée par la séquence de bases SEQ ID NO: 1.


### SEQUENCE LISTING

<110> Sysmex Corporation
<120> Method for judging whether it is a cell of a epithelial cancer origin
<130> TM5427PC
<150> JP 2009-155572
   <151> 2009-06-30
<160> 32
<170> PatentIn version 3.1
<210> 1
   <211> 459
   <212> DNA
   <213> Homo sapiens
<400> 1
   atccatcaac tcagacactg gcgtcctgta tgcactcaga tccttcgact atgagcagtt      60
   tcatgagcta cagatgcagg tgacagccag cgacagcggg gatcctccac tcagcagcaa     120
   cgtgtcgttg agcctgtttg tgctggacca gaacgacaat gcgcccgaga tcctgtaccc     180
   cgccctcccc acagacggtt ccacaggcgt ggagctggcg ccccgctccg cagagcccgg     240
   ctacctggtg accaaggtgg tggcggtgga cagagactcc ggccagaacg cctggctgtc     300
   ctaccgtctg ctcaaggcca gcgagccggg actcttcgcg gtgggggagc acacgggcga     360
   ggtgcgcacg gcgcgagccc tgctggacag agacgcgctc aagcaaagcc tcgtagtggc     420
   cgtccaggac cacggccagc cccctctctc cgccactgt                           459
<210> 2
   <211> 431
   <212> DNA
   <213> Homo sapiens
<400> 2
   aagtggcacg tttgtggcca acttgacaaa ggacctggga ctgagggtgg gggagctggc 60
   ttcgcggggc gctcgggttg ttttcaaagg gaacagacaa catttgcagt ttgatccaca 120
   gacccatgat ttactgctaa atgaaaaact ggaccgggag gagctgtgtg gctccactga 180
   gccgtgtgtg ctacctttcc aagtgttact ggaaaacccc ttgcagtttt ttcaggcttc 240
   cttgcgagtc agagatataa atgaccacgc cccggaattc cctgccagag aaatgctcct 300
   gaaaatatca gaaattacta tgccaggaaa gatatttcct ttgaaaatgg cacacgattt 360
   agacaccggc agcaacggcc ttcagaggta cacaatcagc tccaaccctc acttccacgt 420
   tctcacccgc a 431
<210> 3
   <211> 555
   <212> DNA
   <213> Homo sapiens
<210> 4
   <211> 579
   <212> DNA
   <213> Homo sapiens
<400> 4
   ccgtgcctgt gccacgcgtg tcgccggctg gagagctccg cctggatggt ttcactccgc      60
   ggaggcgccg ccccgccact cagttagaaa cctcagagcc atattcaact ctcgtctcct     120
   ctgcagcacc actgccacag ccctgctttt ctcctcacgt ccaccctcct cgcccccacc     180
   cccgtctcct ggccggtttc cttttctgca gtttgccgcc tccaagccgc cctgcgcttc     240
   ctgcccgtgt ctgacaaaga ctctgctccc ttactgaaca ccacagtgcc cgccacgggc     300
   tgccactcaa ggtcacatcc gcacgaccac cccctgcagc ctacctgcca gtgtcatctc     360
   tggtcacccc tccctcccag gatccatctc ctcatcgtgc ttccgcacct gcggttcatt     420
   ccctgggcca agaatgtccc tcctaattct ttgttcatcc ttagagaccc tgataaatgt     480
   caccacagga gtggaacctt cccctgcccc accccagact tagtctctcc acaacagctt     540
   gcacatcccc ttgtccaaca ttacacttgt ccctacaca                            579
<210> 5
   <211> 26
   <212> DNA
   <213> Artificial
<220>
   <223> sence primer
<400> 5
   gatgtaggtg atagttagcg atagcg 26
<210> 6
   <211> 29
   <212> DNA
   <213> Artificial
<220>
   <223> antisence primer
<400> 6
   cctataaaac cgtctataaa aaaaacgaa 29
<210> 7
   <211> 26
   <212> DNA
   <213> Artificial
<220>
   <223> sence primer
<400> 7
   gaggagttgt gtggttttat tgagtc 26
<210> 8
   <211> 26
   <212> DNA
   <213> Artificial
<220>
   <223> antisence primer
<400> 8
   tctctaacaa aaaattccga aacgta 26
<210> 9
   <211> 28
   <212> DNA
   <213> Artificial
<220>
   <223> sence primer
<400> 9
   ttagaggcga gtaagagtta gggtagtc 28
<210> 10
   <211> 25
   <212> DNA
   <213> Artificial
<220>
   <223> antisence primer
<400> 10
   acctacaaaa acgacacaaa aaacg 25
<210> 11
   <211> 30
   <212> DNA
   <213> Artificial
<220>
   <223> sence primer
<400> 11
   gggttgttat ttaaggttat attcgtacga 30
<210> 12
   <211> 25
   <212> DNA
   <213> Artificial
<220>
   <223> antisence primer
<400> 12
   taaaccgcaa atacgaaaac acgat 25
<210> 13
   <211> 29
   <212> DNA
   <213> Artificial
<220>
   <223> sence primer
<400> 13
   ttttatgagt tatagatgta ggtgatagt 29
<210> 14
   <211> 22
   <212> DNA
   <213> Artificial
<220>
   <223> antisence primer
<400> 14
   ccaccttaat caccaaataa cc 22
<210> 15
   <211> 25
   <212> DNA
   <213> Artificial
<220>
   <223> sence primer
<400> 15
   taaaggattt gggattgagg gtggg 25
<210> 16
   <211> 29
   <212> DNA
   <213> Artificial
<220>
   <223> antisence primer
<400> 16
   ttcaaaaaca tttctctaac aaaaaattc 29
<210> 17
   <211> 26
   <212> DNA
   <213> Artificial
<220>
   <223> sence primer
<400> 17
   gggagttagg tttaggtggg gatatg 26
<210> 18
   <211> 27
   <212> DNA
   <213> Artificial
<220>
   <223> antisence primer
<400> 18
   aaaatcaaaa aacaaaaaac ccttaac 27
<210> 19
   <211> 33
   <212> DNA
   <213> Artificial
<220>
   <223> sence primer
<400> 19
   ttgtagtatt attgttatag ttttgttttt ttt 33
<210> 20
   <211> 35
   <212> DNA
   <213> Artificial
<220>
   <223> antisence primer
<400> 20
   attccactcc tataataaca tttatcaaaa tctct 35
<210> 21
   <211> 18
   <212> DNA
   <213> Artificial
<220>
   <223> GSTP1 sence primer
<400> 21
   gaggccttcg ctggagtt 18
<210> 22
   <211> 20
   <212> DNA
   <213> Artificial
<220>
   <223> GSTP1 antisence primer
<400> 22
   gtactcactg gtggcgaaga 20
<210> 23
   <211> 20
   <212> DNA
   <213> Artificial
<220>
   <223> CDH1 sence primer
<400> 23
   gtgaaccctc agccaatcag 20
<210> 24
   <211> 18
   <212> DNA
   <213> Artificial
<220>
   <223> CDH1 antisence primer
<400> 24
   agttccgacg ccactgag 18
<210> 25
   <211> 19
   <212> DNA
   <213> Artificial
<220>
   <223> ER sence primer
<400> 25
   gcctacgagt tcaacgccg 19
<210> 26
   <211> 18
   <212> DNA
   <213> Artificial
<220>
   <223> ER antisence primer
<400> 26
   aacgccgcag cctcagac 18
<210> 27
   <211> 25
   <212> DNA
   <213> Artificial
<220>
   <223> ch14-cgf1 sence primer
<400> 27
   ggaggagtca agagaagttg gaagc 25
<210> 28
   <211> 23
   <212> DNA
   <213> Artificial
<220>
   <223> ch14-cgf1 antisence primer
<400> 28
   cccacactcc atttccattc ctc 23
<210> 29
   <211> 25
   <212> DNA
   <213> Artificial
<220>
   <223> ch14-cgf2 sence primer
<400> 29
   gggtactttg ccaatatagc catgc 25
<210> 30
   <211> 23
   <212> DNA
   <213> Artificial
<220>
   <223> ch14-cgf2 antisence primer
<400> 30
   tggctaagtg ggagggagaa cag 23
<210> 31
   <211> 21
   <212> DNA
   <213> Artificial
<220>
   <223> ch14-cgf3 sence primer
<400> 31
   ggatgggaga cacctggttc a 21
<210> 32
   <211> 24
   <212> DNA
   <213> Artificial
<220>
   <223> ch14-cgf3 antisence primer
<400> 32
   ggatggacca gctgctttgt actc 24